# EUROPEAN PATENT APPLICATION

(11) **EP 3 674 311 A1**
(43) Date of publication of application: **01.07.2020**
(21) Application number: 18847695.6
(22) Date of filing: 23.08.2018
(51) Int. Cl.: C07K 5/037, A61K 31/704, A61K 31/7048, A61K 47/64, A61P 31/04, A61P 31/10, A61P 35/00, C07K 5/068, C07K 5/09, C07K 5/11, C07K 7/00, C07K 14/00, C08G 69/26, C12P 13/04

(54) **E-POLY-L-LYSINE DERIVATIVE HAVING CLICK FUNCTIONAL GROUP, METHOD FOR PRODUCING SAME, AND USE THEREOF**

(30) Priority: 23.08.2017 JP 2017159848; 23.08.2017 JP 2017159849
(71) Applicant: Fukui Prefectural University, Yoshida-gun, Fukui 910-1195 (JP)
(72) Inventor: HAMANO, Yoshimitsu, Yoshida-gun Fukui 910-1195 (JP); USHIMARU, Kazunori, Yoshida-gun Fukui 910-1195 (JP)
(74) Representative: Teipel, Stephan
(86) International application number: PCT/JP2018/031153
(87) International publication number: WO 2019/039544

(57) **Abstract**

The present invention provides a compound having a structure represented by the general formula (1): 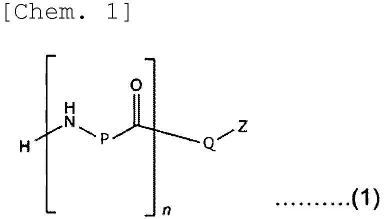 (wherein
n is 2 to 100;
P represents a (C₁-C₅) alkylene group substituted with one amino group;
Q is represented by the following general formula (2): 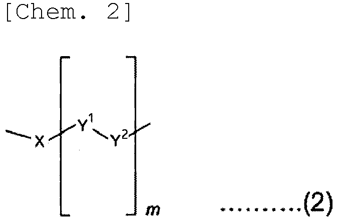 (wherein X represents an oxygen atom, Y¹ and Y² each represent an alkylene group of 1 to 6 carbon atoms, and m is an integer of 1 to 30); and
Z represents a click functional group) or
a salt thereof and also provides a pharmacological substance which has the compound or a salt thereof attached thereto by a chemical bond and has an improved biomembrane permeability as well as an improved water solubility.

## Description

### TECHNICAL FIELD

The present invention relates to ε-poly-L-lysine derivatives having a carboxyl terminal modified with a functional group for click chemistry, a method for producing the same, and use thereof for improvement in biomembrane permeability as well as water solubility of pharmacological substances, polycationic modification of industrial materials, and antimicrobial coating of industrial materials.

### BACKGROUND ART

ε-poly-L-lysine (hereinafter referred to as ε-PL) is an amino acid homopolymer consisting of a linear chain of 10 to 35 proteinogenic amino acid L-lysine residues. In the linear chain structure, isopeptide bonds are formed between the carboxyl group of one L-lysine residue and the amino group of the adjacent one. ε-PL is produced by several kinds of microbes including actinomycetes, and its peptide chain length (the number of L-lysine residues) varies with the kind of the ε-PL-producing microbe (Non Patent Literature 1 to 4). The first reported ε-PL was an ε-PL consisting of 25 to 35 residues identified from the culture of an actinomycete, *Streptomyces albulus* strain No. 346-D. After the first report, an ε-PL having a shorter chain than that of the first reported ε-PL (short-chainε-PL) was identified. However, no ε-PL consisting of 36 or more L-lysine residues has been found in nature.

The α-amino group (-NH₂) in ε-PLs is protonated (-NH₃⁺), for example, under acidic to neutral pH conditions in the body. Thus, ε-PLs are present as super water-soluble polycationic peptides and exert antimicrobial activity against a wide spectrum of microbes, which activity is comparable to those of synthetic polycationic antibiotics (Non Patent Literature 4) . The 25- to 35-mer ε-PL produced by *Streptomyces albulus* strain No. 346-D is used as a food antiseptic or a food preservative due to its wide-spectrum antimicrobial activity and high safety. This ε-PL is also expected as a promising biodegradable natural antimicrobial agent with high safety and low environmental impact in the fields of chemical industry, medicine, and others as well as food industry.

The present inventors noted the fact that polycationic compounds not only have antimicrobial activity, but also have high biomembrane permeability in spite of their high polarity. The present inventors thought that conjugation of ε-PL to pharmacological substances (hereinafter referred to as ε-PL modification) would improve the water solubility and biomembrane permeability of the pharmacological substances (Non Patent Literature 4). The present inventors also thought that ε-PL modification of the surface of various materials for medical, manufacturing, or other industrial uses (hereinafter collectively referred to as industrial materials), such as plastics, metal, wood, paper, and textiles, would provide such materials with various preferable functions based on the polycationic nature of ε-PL, such as antimicrobial activity, cultured cell adhesive function, drip proofness, and antistatic function. There has been no report on ε-PL modification of pharmacological substances. ε-PL modification of industrial materials was previously reported, which is a technique based on the electrostatic interaction of the polycationic nature (positive charge) of ε-PL and the surface negative charge of the industrial materials (Patent Literature 1). However, this chemical modification technique using noncovalent bonding is very inferior in terms of stability and durability, and is not yet an established ε-PL modification technique for practical use. In addition, this modification technique can be applied only to materials with a surface negative charge and cannot be said to be a versatile modification technique. Further, the ε-PL modification of industrial materials using the electrostatic interaction results in reduction in the polycationic nature (positive charge) of ε-PL, which is a fundamental chemical property related to antimicrobial activity, cultured cell adhesive function, drip proofness, antistatic function, etc. Thus, this modification technique cannot provide sufficient functions as expected. The present inventors developed an ε-PL modification technique using covalent bonding instead of the electrostatic interaction and found that the technique can produce potent effects.

One of the known methods for ε-PL production is a method using *Streptomyces albulus* strain No. 346-D (NBRC14147) isolated from nature (Patent Literature 2). Also known are ε-PL production methods using species other than *Streptomyces albulus,* for example, *Streptomyces noursei* (Patent Literature 3) and *Streptomyces* sp. strain SP-72 (Patent Literature 4). In addition, a short-chain ε-PL production method using *Streptomyces* sp. strain SP-66 (Patent Literature 5) is known.

For chemical modification of pharmacological substances and industrial materials using a more stable covalent bond without sacrificing the polycationic nature of ε-PL in any way, synthetic organic chemistry using the carboxyl group which is only one present in one ε-PL molecule is required. To this end, a high-level synthetic organic chemistry technique for protecting all the α amino groups (-NH₂) of the ε-PL with protective groups is required, but there have been no successful reports.

Click chemistry is an organic synthesis technique using a functional group with high reaction specificity (hereinafter referred to as a click functional group) without use of any protective group and is used not only in the chemical industry field but also in other fields. If a click functional group could be attached to the carboxyl group of ε-PL, it would be possible to easily perform ε-PL modification of pharmacological substances and industrial materials. Even for attaching a click functional group to the carboxyl group of ε-PL, a high-level synthetic organic chemistry technique for protecting all the α amino groups (-NH₂) of the ε-PL with protective groups is required, and there have been no successful reports. There are also no reports on the production of ε-PL derivatives in which a click functional group is attached to ε-PL in ε-PL-producing microbes.

### CITATION LIST

### Patent Literature

Patent Literature 1: Japanese Patent No. 3111216
Patent Literature 2: JP-B 59-20359
Patent Literature 3: JP-A 1-187090
Patent Literature 4: JP-A 2000-69988
Patent Literature 5: JP-A 2001-017159

### Non Patent Literature

Non Patent Literature 1:
   Shima S, Sakai H. 1977. Polylysine produced by Streptomyces. 1977.
Non Patent Literature 2:
   Oppermann-Sanio FB, Steinbuechel A. 2002. Occurrence, functions and biosynthesis of polyamides in microorganisms and biotechnological production. 2002.
Non Patent Literature 3:
   TakeharaM, Hirohara H. 2010. Amino-acid homopolymersoccurring in nature, p. 1-22. In Hamano Y (ed.), Microbiology Monographs, vol. 15. Springer, New York.
Non Patent Literature 4:
   Hamano Y. 2010. Amino-acid homopolymers occurring in nature: Biochemistry and enzymology of poly-ε-L-lysine, p. 23-44. In Hamano (ed), Microbiology Monographs, vol. 15. Springer, New York.

### SUMMARY OF INVENTION

### TECHNICAL PROBLEM

An object of the present invention is to provide a compound having a structure represented by the general formula (1): 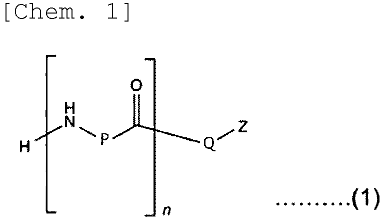 (wherein
n is 2 to 100;
P represents a (C₁-C₅) alkylene group substituted by an amino group;
Q is a structure represented by the following general formula (2): 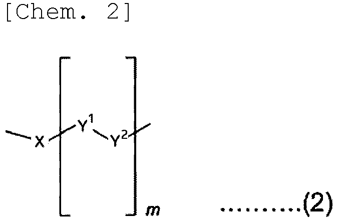 (wherein X represents an oxygen atom, an imino group, a sulfur atom, NHCO, or CONH; Y¹ and Y² may be the same or different and each represent an alkylene group of 1 to 6 carbon atoms; and m is an integer of 1 to 30) or
   the following general formula (3): 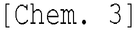 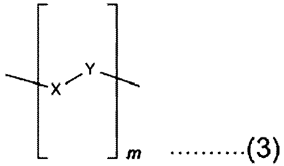 (wherein X represents an oxygen atom, an imino group, a sulfur atom, NHCO, or CONH; Y represents an alkylene group of 1 to 6 carbon atoms; and m is an integer of 1 to 30); and
Z represents a click functional group) or
   a chemically or pharmaceutically acceptable salt thereof and to provide a method for producing the compound or the chemically or pharmaceutically acceptable salt thereof (hereinafter, the chemically acceptable salt and the pharmaceutically acceptable salt may be collectively referred to simply as the salt).

Another object of the present invention is to provide an ε-poly-L-lysine (ε-PL) derivative having a structure represented by the general formula (4): 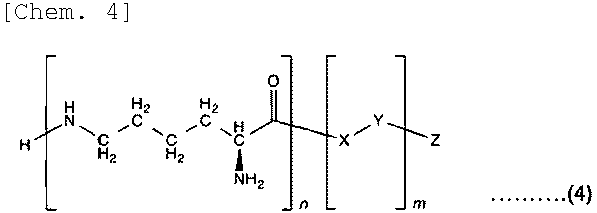 (wherein
n is 2 to 100;
m is an integer of 1 to 30;
X represents an oxygen atom or an imino group;
Y represents an alkylene group of 1 to 6 carbon atoms; and
Z represents a click functional group) or
   a chemically or pharmaceutically acceptable salt thereof and to provide a method for producing the compound or the chemically or pharmaceutically acceptable salt thereof (hereinafter, the chemically acceptable salt and the pharmaceutically acceptable salt may be collectively referred to simply as the salt).

Yet another object of the present invention is to provide a modified pharmacological substance in which the above compound (including the ε-PL derivative) is covalently attached to an original pharmacological substance which is, for example, not only a low- or medium-molecular compound such as an antimicrobial or anticancer substance, but also a high-molecular compound such as a protein (including an enzyme), wherein the modified pharmacological agent has an improved biomembrane permeability as well as an improved water solubility, and to provide a method for producing the same. Still another object of the present invention is to provide use of the above compound (including the ε-PL derivative) for improvement of biomembrane permeability of pharmacological substances.

Furthermore, the above compound (including the ε-PL derivative) is intended for use in attachment to the surface of various industrial materials by a covalent bond, providing the industrial materials with various functions based on the polycationic nature, such as antimicrobial activity, cultured cell adhesive function, drip proofness, and antistatic function.

### SOLUTION TO PROBLEM

The present invention includes the following to achieve the above-mentioned objects.
[1] A compound having a structure represented by the general formula (1): 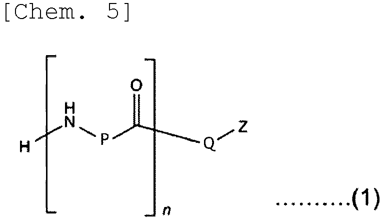 (wherein
   n is 2 to 100;
   P represents a (C₁-C₅) alkylene group substituted by an amino group;
   Q is a structure represented by the following general formula (2): 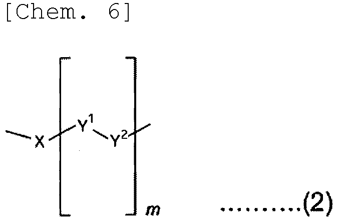 (wherein X represents an oxygen atom, an imino group, a sulfur atom, NHCO, or CONH; Y¹ and Y² may be the same or different and each represent an alkylene group of 1 to 6 carbon atoms, an oxygen atom, or an imino group; and m is an integer of 1 to 30) or the following general formula (3): 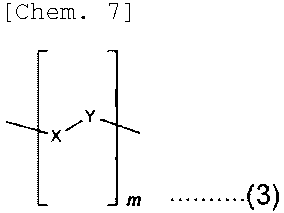 (wherein X represents an oxygen atom, an imino group, a sulfur atom, NHCO, or CONH; Y represents an alkylene group of 1 to 6 carbon atoms, an oxygen atom, or an imino group; and m is an integer of 1 to 30); and
   Z represents a click functional group) or
      a salt thereof.
[2] The compound or the salt thereof according to the above [1], wherein the compound is represented by the general formula (4) : 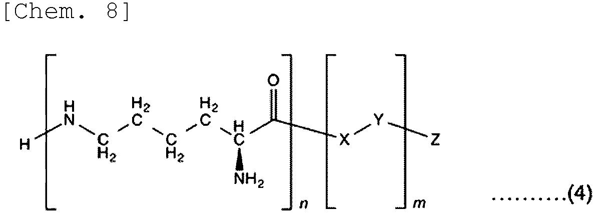 (wherein
   - n: is an integer of 2 to 100;
   - m: is an integer of 1 to 30;
   - X: represents an oxygen atom or an imino group;
   - Y: represents an alkylene group of 1 to 6 carbon atoms; and
   - Z: represents a click functional group).
[3] The compound or the salt thereof according to the above [1] or [2], wherein the click functional group is an azido group, an alkenyl group, an alkynyl group, a nitrile group, a thiol group, a maleimide group, an epoxide group, an aziridine group, a thiirane group, or a triazole group.
[4] A method for producing the compound or the salt thereof according to the above [1], the method comprising the steps of:
   culturing a microbe capable of producing ε-poly-L-lysine (ε-PL), ε-poly-D-lysine, ε-poly-L-β-lysine, ε-poly-D-β-lysine, δ-poly-L-ornithine, δ-poly-D-ornithine, δ-poly-L-β-ornithine, δ-poly-D-β-ornithine, γ-poly-L-diaminobutanoic acid, γ-poly-D-diaminobutanoic acid, β-poly-L-diaminopropionic acid, or β-poly-D-diaminopropionic acid, wherein the culture is performed in the presence of a compound represented by the general formula (2a): 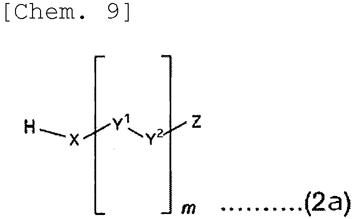 (wherein X, Y¹, Y², Z, and m are as defined in the above general formula (1)) or
   the general formula (3a): 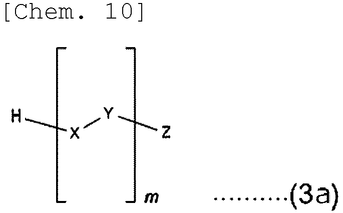 (wherein X, Y, Z, and m are as defined in the above general formula (1)) or
   a salt thereof; and
   harvesting the compound or the salt thereof according to the above [1] produced in the previous step.
[5] A method for producing the compound or the salt thereof according to the above [2], the method comprising the steps of:
   culturing a microbe capable of producing ε-PL, wherein the culture is performed in the presence of a compound represented by the general formula (3a): 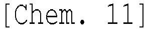 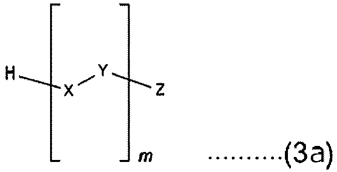 (wherein X represents an oxygen atom or an imino group, Y represents an alkylene group of 1 to 6 carbon atoms, Z represents a click functional group, and m represents an integer of 1 to 30) or
   a salt thereof; and
   harvesting the compound or the salt thereof according to the above [2] produced in the previous step.
[6] A pharmacological substance having the compound according to the above [1] or a pharmaceutically acceptable salt thereof attached thereto by a chemical bond.
[7] A pharmacological substance having a compound represented by the general formula (4): 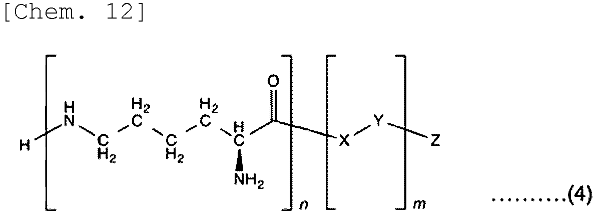 (wherein
   n is an integer of 2 to 100;
   m is an integer of 1 to 30;
   X represents an oxygen atom and an imino group;
   Y represents an alkylene group of 1 to 6 carbon atoms; and
   Z represents a click functional group) or
      a pharmaceutically acceptable salt thereof attached thereto by a chemical bond.
[8] The substance according to the above [6] or [7], wherein the pharmacological substance is an antimicrobial agent, an antibacterial agent, or an anticancer agent.
[9] The substance according to the above [8],
   wherein the antimicrobial agent is (A) an antifungal agent;
   wherein the antibacterial agent is (B) a β lactam antibiotic, (C) an aminoglycoside antibiotic, (D) a tetracycline antibiotic, (E) a peptide antibiotic, or (F) an antibacterial antibiotic that can form a bond with the click functional group through an organic chemical or biochemical process; or
   wherein the anticancer agent is (G) an anticancer agent that can form a bond with the click functional group through an organic chemical or biochemical process.
[10] The substance according to the above [9],
   wherein (A) the antifungal agent is (a-1) a polyene antibiotic selected from amphotericin B (hereinafter referred to as AmpB), nystatin, trichomycin, and pimaricin; (a-2) micafungin; (a-3) itraconazole; (a-4) ketoconazole; (a-5) fluconazole; (a-6) miconazole; or (a-7) flucytosine,
   wherein (B) the β lactam antibiotic is (b-1) penicillin; (b-2) ampicillin; (b-3) talampicillin; (b-4) bacampicillin; (b-5) cephalosporin C; (b-6) cefalexin; (b-7) cefradine; (b-8) cephamycin; or (b-9) imipenem,
   wherein (C) the aminoglycoside antibiotic is (c-1) gentamicin; (c-2) kanamycin; (c-3) tobramycin; (c-4) amikacin; (c-5) arbekacin; or (c-6) ribostamycin,
   wherein (D) the tetracycline antibiotic is (d-1) oxytetracycline; or (d-2) tetracycline,
   wherein (E) the peptide antibiotic is (e-1) bacitracin; (e-2) gramicidin; (e-3) polymyxin B; (e-4) vancomycin; or (e-5) teicoplanin,
   wherein (F) the antibacterial antibiotic that can form a bond with the click functional group through an organic chemical or biochemical process is an antibacterial antibiotic having an amino group, an imino group, a carboxyl group, a hydroxy group, a carbonyl group, a thiol group, a phosphate group, an aldehyde group, or the like, or
   wherein (G) the anticancer agent that can form a bond with the click functional group through an organic chemical or biochemical process is an anticancer agent having an amino group, an imino group, a carboxyl group, a hydroxy group, a carbonyl group, a thiol group, a phosphate group, an aldehyde group, or the like, for example, (f-1) actinomycin D; (f-2) mitomycin; (f-3) an anthracycline antineoplastic agent selected from doxorubicin (hereinafter referred to as Dox), daunorubicin, and aclarubicin; (f-4) bleomycin; or (f-5) cisplatin.
[11] The substance according to the above [6] or [7], wherein the pharmacological substance is a protein such as an enzyme or an antibody, or a gene.
[12] The substance according to the above [11], wherein the protein or gene is a protein or gene that can form a bond with the click functional group through an organic chemical or biochemical process.
[13] A method for producing the pharmacological substance according to the above [7], the method comprising attaching the compound represented by the general formula (4) or the pharmaceutically acceptable salt thereof specified in the above [7] to a pharmacological substance by a chemical bond.
[14] The substance according to any one of the above [7] to [10], wherein the substance is an AmpB modified with ε-PL by click chemistry (hereinafter referred to as ε-PL-DBCO-AmpB) and is represented by the general formula (5): 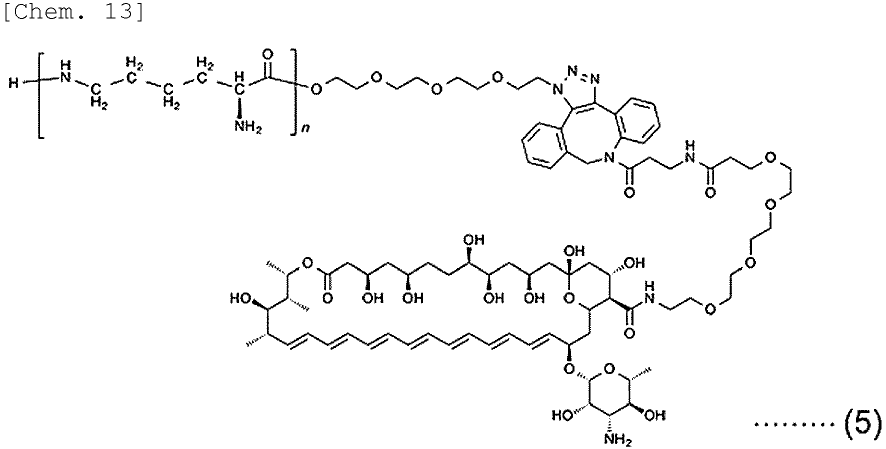 (wherein n is an integer of 2 to 100).
[15] The substance according to any one of the above [7] to [10], wherein the substance is a Dox modified with ε-PL by click chemistry (hereinafter referred to as ε-PL-DBCO-Dox) and is represented by the general formula (5-2): 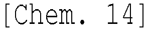 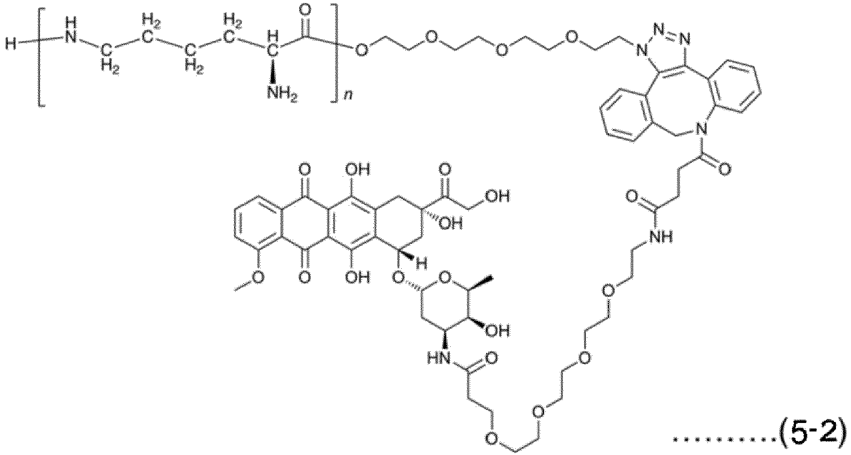 (wherein n is an integer of 2 to 100).
[16] The substance according to any one of the above [7] to [10], wherein the substance is a fluorescent protein monomeric Azami-Green modified with ε-PL by click chemistry (hereinafter referred to as ε-PL-DBCO-mAG) and is represented by the general formula (5-3): 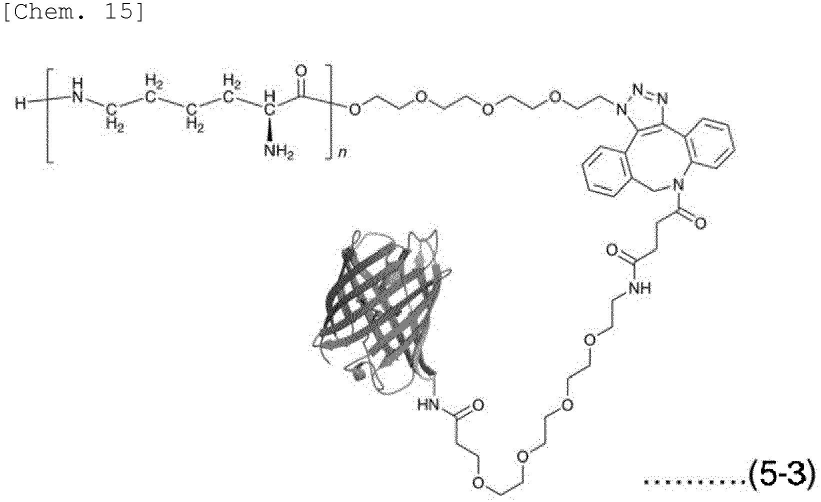 (wherein n is an integer of 2 to 100, and
   a group represented by formula (5-3)': 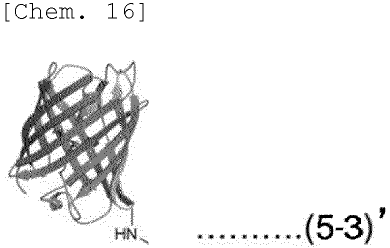 is formed by removing one hydrogen atom from a fluorescent protein monomeric Azami-Green (hereinafter referred to as mAG) represented by formula (5-3)": 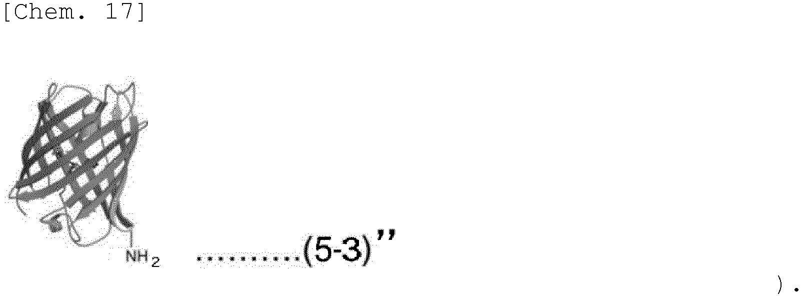
[17] Use of a compound represented by the general formula (2a) or (3a) or a salt thereof for production of the compound represented by the general formula (1) or the salt thereof according to the above [1].
[18] Use of a compound represented by the general formula (3a) : 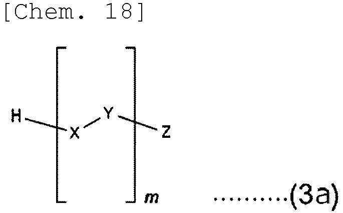 (wherein X represents an oxygen atom or an imino group, Y represents an alkylene group of 1 to 6 carbon atoms, Z represents a click functional group, and m represents an integer of 1 to 30) or
   a salt thereof for production of the compound represented by the general formula (4) or the salt thereof specified in the above [7].
[19] A method for improving biomembrane permeability of a pharmacological substance, the method comprising attaching a compound represented by the general formula (1) or a salt thereof to the pharmacological substance, or use of the compound represented by the general formula (1) or the salt thereof for production of a pharmacological substance having an improved biomembrane permeability.
[20] A method for improving biomembrane permeability of a pharmacological substance, the method comprising attaching the compound represented by the general formula (4) or the salt thereof specified in the above [7] to the pharmacological substance, or use of the compound represented by the general formula (4) or the salt thereof for production of a pharmacological substance having an improved biomembrane permeability.
[21] A method for improving water solubility of a pharmacological substance, the method comprising attaching a compound represented by the general formula (1) or a salt thereof to the pharmacological substance.
[22] Use of a compound represented by the general formula (1) or a salt thereof for production of a pharmacological substance having an improved water solubility.
[23] A method for improving water solubility of a pharmacological substance, the method comprising attaching the compound represented by the general formula (4) or the salt thereof specified in the above [7] to the pharmacological substance, or use of the compound represented by the general formula (4) for production of a pharmacological substance having an improved water solubility.

### ADVANTAGEOUS EFFECTS OF INVENTION

Enhancement of drug excretory function in pathogenic microbes and cancer cells leads to the resistance to drugs for treatment. An effective strategy for improving the biomembrane permeability of pharmacological substances such as drugs would not only overcome drug resistance, but also allow reuse of pharmacological substances that have been rendered unusable due to resistance and increase in the bioactivity of the pharmacological substances. A general strategy for improving the membrane permeability of pharmacological substances is hydrophobization. However, this strategy inevitably reduces the water solubility of the pharmacological substances, resulting in new problems, such as unexpected adverse effects, complicated formulation preparation, etc. The present inventors thought that the development of chemical modification techniques for improving the biomembrane permeability as well as water solubility of low-molecular functional compounds would further accelerate drug development as desired in the modern society.

High-molecular compounds such as proteins (including enzymes) do not usually permeate cell membranes. This nature is essential for the maintenance of cellular homeostasis, but makes it difficult to directly introduce such high molecules into the cell from the outside for the purpose of functional analysis, functional regulation, or medical treatment. Conventional techniques for intracellular introduction of substances include microinjection and electroporation, which involve temporal disruption of the cell membrane, and liposome-based transfection. However, the results are not always satisfactory due to variation in transfection efficiency and heavy damage to cells. Meanwhile, a lot of attention has been attracted to high cell permeability of basic (polycationic) peptides, and various studies have been actively conducted on direct intracellular introduction of proteins (including enzymes) modified with polycationic peptides (Non Patent Literature, S.R. Schwarze, et al., Science, 285, 1569, 1999). There are actually a large number of reports that revealed successful regulation of cell function only by adding, to cell culture medium, of polycation-modified proteins prepared by chemical bridging or fusion with polycationic peptides. Polycationic modification has now been recognized as one of the cell biochemical techniques and has also attracted attention as a novel method for delivering biomedicines (antibody drugs etc.).

The present inventors conceived that the above problems can be solved by polycationic compounds including ε-PL, which is a natural polycationic compound having high polarity, super water solubility, and high biomembrane permeability. On the bacterial cell membrane surface, acidic phospholipids are exposed, and the animal cell surface is abundant in sugar chains containing a number of sulfuric acid groups and carboxyl groups. Thus, both cell surfaces are negatively charged. The present inventors thought that polycationic compounds with positive charge (including ε-PL) would be drawn to the negatively charged cell surface, easily permeate the cell membrane, and enter the cell. That is, the polycationic compound used in the present disclosure means, for example, a compound having polycationic nature. The polycationic compound can be generated by, for example, attachment of H⁺ to the amino group of each lysine residue of a polylysine, for example, in the living body.

In addition, natural polycationic compounds similar to ε-PL, such as γ-poly-L-diaminobutanoic acid, γ-poly-D-diaminobutanoic acid, and β-poly-L-diaminopropionic acid, can produce the same effect as that of ε-PL.

The present inventors found that the compound represented by the general formula (1) described in the above [1] (including the ε-PL derivative) can be easily conjugated to various pharmacological substances via a stable covalent bond by click chemistry. In general, highly water-soluble compounds have low biomembrane permeability, but pharmacological substances having the polycationic compound (including ε-PL) attached thereto by a covalent bond remain very highly water soluble and have very high biomembrane permeability due to the polycationic nature of the polycationic compound (including ε-PL). Thus, such a simple polycationic (including ε-PL) modification method can improve the biomembrane permeability as well as water solubility of pharmacological substances, and a novel, simple and effective method for producing a compound required for the polycationic modification method is one of the essential features of the present invention.

Even high-molecular compounds such as proteins (including enzymes) can be rendered permeable to biomembranes through polycationic modification by click chemistry. The present disclosure provides a novel method for producing a high-molecular compound that can be directly delivered into a cell. Click chemistry is a well-established technique and can be performed in a well-established manner in the present invention.

According to the present invention, polycationic modification (including ε-PL modification) of various industrial materials (e.g., medical devices and functional materials made of plastics, textiles, etc.) etc. can be performed by treating such materials with a pharmacological substance having the above-mentioned compound represented by the general formula (1) (including the compound represented by the general formula (4) herein) or a salt thereof attached thereto. As a result, excellent antimicrobial activity, cultured cell adhesive function, hydrophilicity, drip proofness, and antistatic function can be provided to the industrial materials.

### BRIEF DESCRIPTION OF DRAWINGS

Fig. 1A shows the chromatogram from ESI-TOF-MS/HPLC analysis of the culture supernatant of *Streptomyces albulus* NBRC14147. The top trace represents the analytical result of the non-supplemented culture broth. The middle trace represents the analytical result of the culture broth in the presence of ε-PL-PEG-azide. The bottom trace represents the analytical result of the culture broth in the presence of ε-PL-PEG-alkyne.
Fig. 1B shows the results of ESI-TOF-MS analysis of ε-PL (top chart), ε-PL-PEG-azide (middle chart), and s-PL-PEG-alkyne (bottom chart).
Fig. 2A shows the results of ¹H-NMR structural determination of a 17- to 26-mer ε-PL-PEG-azide produced by the ε-PL-producing microbe *Streptomyces albulus* NBRC14147.
Fig. 2B shows the results of ¹H-NMR structural determination of an ε-PL-PEG-alkyne produced by the ε-PL-producing microbe *Streptomyces albulus* NBRC14147.
Fig. 3A shows the chromatogram from ESI-TOF-MS/HPLC analysis of the reaction mixture containing an ε-PL-DBCO-FAM synthesized by click chemistry.
Fig. 3B shows the results of ESI-TOF-MS analysis of the synthesized ε-PL-DBCO-FAM.
Fig. 4 shows the results of testing the biomembrane permeability of the DBCO-PEG₄-5/6-FAM represented by formula (12) and the ε-PL-DBCO-FAM represented by the general formula (13) using HeLa cells.
Fig. 5 shows the confocal laser scanning microscopic results of evaluating the biomembrane permeability of the ε-PL-DBCO-FAM represented by the general formula (13) using HeLa cells. The biomembrane and nucleus were stained with Alexa594 WGA (wheat germ lectin) and 4',6-diamidino-2-phenylindole (DAPI), respectively.
Fig. 6A shows the chromatogram from ESI-TOF-MS/HPLC analysis of the reaction mixture containing an ε-PL-DBCO-AmpB synthesized by click chemistry.
Fig. 6B shows the results of ESI-TOF-MS analysis of the synthesized ε-PL-DBCO-AmpB.
Fig. 7 shows the water solubility of ε-PL-DBCO-AmpB.
Fig. 8A shows the chromatogram from ESI-TOF-MS/HPLC analysis of the reaction mixture containing an ε-PL-DBCO-Dox synthesized by click chemistry.
Fig. 8B shows the results of ESI-TOF-MS analysis of the synthesized ε-PL-DBCO-Dox.
Fig. 9A shows the chromatogram from ESI-TOF-MS/HPLC analysis of the mAG represented by general formula (5-3)" (left), the mass spectrum from ESI-TOF-MS analysis of the mAG (center), and the results of deconvolution of the obtained mass spectrum (right).
Fig. 9B shows the chromatogram from ESI-TOF-MS/HPLC analysis of DBCO-mAG (left), the mass spectrum from ESI-TOF-MS analysis of DBCO-mAG (center), and the results of deconvolution of the obtained mass spectrum (right).
Fig. 9C shows the chromatogram from ESI-TOF-MS/HPLC analysis of the ε-PL-DBCO-mAG represented by the general formula (5-3) (left), the mass spectrum from ESI-TOF-MS analysis of the ε-PL-DBCO-mAG (center), and the results of deconvolution of the obtained mass spectrum (right).
Fig. 10 shows the results of testing the biomembrane permeability of the mAG represented by the general formula (5-3)'' and the ε-PL-DBCO-mAG represented by the general formula (5-3) using HeLa cells.
Fig. 11 shows the confocal laser scanning microscopic results of evaluating the biomembrane permeability of the ε-PL-DBCO-mAG represented by the general formula (5-3) using HeLa cells. The biomembrane was stained with Alexa594 WGA (wheat germ lectin).
Fig. 12A shows the results of the examination of the chemical stability of the ester bond of the ε-PL-alkyne represented by the general formula (15). ε-PL-alkyne was dissolved in Tris-HCl buffer solution (pH 8.0) at a concentration of 1 mM, heated to 60°C for 5 minutes, and analyzed by ESI-TOF-MS/HPLC. # represents the signal from ε-PL-alkyne, and * represents the signal from ε-PL generated by hydrolysis of the ester bond of ε-PL-alkyne.
Fig. 12B shows the results of the examination of the chemical stability of the ester bond of the ε-PL-PEG-alkyne represented by the general formula (11). ε-PL-PEG-alkyne was dissolved in Tris-HCl buffer solution (pH 8.0) at a concentration of 1 mM, heated to 60°C for 5 minutes, and analyzed by ESI-TOF-MS/HPLC. + represents the signal from ε-PL-PEG-alkyne, and * represents the signal from ε-PL generated by hydrolysis of the ester bond of ε-PL-PEG-alkyne.

### DESCRIPTION OF EMBODIMENTS

In the present invention, P in the compound represented by the general formula (1) is a (C₁-C₅) alkylene group substituted by one or more amino groups. The (C₁-C₅) alkylene group may be a straight or branched chain alkylene group, but is preferably a straight chain alkylene group. P in the compound represented by the general formula (1) is preferably a (C₁-C₅) alkylene group substituted by one amino group, and specific examples include CH₂CH₂CH₂CH₂C*H(NH₂), CH₂CH₂CH₂C*H(NH₂)CH₂, CH₂CH₂C*H(NH₂)CH₂CH₂, CH₂C*H(NH₂)CH₂CH₂CH₂, C*H(NH₂)CH₂CH₂CH₂CH₂, CH₂CH₂CH₂C*H(NH₂), CH₂CH₂C*H(NH₂)CH₂, CH₂C*H(NH₂)CH₂CH₂, C*H(NH₂)CH₂CH₂CH₂, CH₂CH₂C*H(NH₂), CH₂C*H(NH₂)CH₂, C*H(NH₂)CH₂CH₂, CH₂C*H(NH₂), and C*H(NH₂)CH₂. The C* represents a chiral carbon and may be an R or S configuration.

In the compound represented by the general formula (1) in the present invention, the imino group represented by X is expressed as -NR-, and R may be H or a suitable substituent such as a lower alkyl group of 1 to 6 carbon atoms (e.g., a methyl group, an ethyl group, etc.), an aryl group (e.g., a phenyl group etc.), or a heterocyclic group containing 1 to 3 heteroatoms (e.g., a furyl group, a pyridyl group, a thienyl group, etc.). Particularly, R is preferably a 5- to 6-membered heterocyclic group.

The alkylene group of 1 to 6 carbon atoms represented by Y, Y¹, or Y² may be, for example, a methylene group, an ethylene group, a propylene group, an isopropylene group, a butylene group, an isobutylene group, a pentene group, a hexene group, or the like. Particularly, Y is preferably an alkylene group of 2 to 6 carbon atoms. The alkylene group may be substituted by an ordinary substituent, and such an substituent may be, for example, a halogen atom (e.g., a bromine atom, a chlorine atom, a fluorine atom, etc.), a hydroxyl group, an amino group, a mercapto group, a carboxyl group, a sulfo group, or the like. In the alkylene group, an intervening group, such as -O-, -S-, -S-S-, or a silyl group, may be contained.

The click functional group in the present invention may be, for example, an azido group (-N₃), an alkenyl group (e.g., -CH=CH₂, -CH=CH-CH₃, etc.), an alkynyl group (e.g., -C=CH, -C≡C-CH₃, etc.), a nitrile group, a thiol group, a maleimide group, an epoxide group, an aziridine group, a thiirane group, a triazole group, or the like.

In the general formulae (1), (4), (5), (5-2), and (5-3) and the general formulae (6), (7), (10), (11), (13), and (15) shown later, n may be any of 2 to 100, but is preferably about 10 to 40, more preferably about 9 to 30. n varies with the type of the microbe to be cultured, and the culture conditions (e.g., pH, temperature, time, etc.) of the microbe and cannot be definitely specified, but usually preferred is a compound in which n is 2 to 35, a compound in which n is 25 to 35, a compound in which n is 9 to 18, or a compound in which n is 17 to 26.

In the general formula (1) or (4), m may be any of 1 to 30, but is preferably about 2 to 20. When m is 2 or more, the attachment of the compound represented by the general formula (1) or a salt thereof to a pharmacological substance results in more improvement in biomembrane permeability, water solubility and chemical stability of the pharmacological substance.

Specific examples of the compound represented by the general formula (1) (in the case where Q is a structure represented by the general formula (2)) are shown below.

**[Table 1]**

| Compound No. | X | Y¹ | Y² | Z | m | n |
|---|---|---|---|---|---|---|
| 1 | O | CH₂ | O | N₃ | 4 | 30 |
| 2 | O | CH₂CH₂ | NH | N₃ | 12 | 10 |
| 3 | O | CH₂CH₂CH₂ | NCH₃ | N₃ | 4 | 20 |
| 4 | O | CH₂CH₂CH₂CH₂ | O | N₃ | 4 | 40 |
| 5 | O | CH₂CH₂CH₂CH₂CH₂ | NH | N₃ | 4 | 50 |
| 6 | O | CH₂CH₂CH₂CH₂CH₂CH₂ | NCH₃ | N₃ | 2 | 60 |
| 7 | NH | CH₂ | O | N₃ | 30 | 70 |
| 8 | NH | CH₂CH₂ | NH | N₃ | 10 | 80 |
| 9 | NH | CH₂CH₂CH₂ | NCH₃ | N₃ | 4 | 90 |
| 10 | NH | CH₂CH₂CH₂CH₂ | O | N₃ | 4 | 100 |
| 11 | NH | CH₂CH₂CH₂CH₂CH₂ | NH | N₃ | 4 | 2 |
| 12 | NH | CH₂CH₂CH₂CH₂CH₂CH₂ | NCH₃ | N₃ | 4 | 25 |
| 13 | NCH₃ | CH₂ | O | N₃ | 20 | 30 |
| 14 | NCH₃ | CH₂CH₂ | NH | N₃ | 8 | 35 |
| 15 | NCH₃ | CH₂CH₂CH₂ | NCH₃ | N₃ | 4 | 25 |
| 16 | NCH₃ | CH₂CH₂CH₂CH₂ | O | N₃ | 4 | 30 |
| 17 | NCH₃ | CH₂CH₂CH₂CH₂CH₂ | NH | N₃ | 4 | 35 |
| 18 | NCH₃ | CH₂CH₂CH₂CH₂CH₂CH₂ | NCH₃ | N₃ | 4 | 25 |
| 19 | NHCO | CH₂ | O | N₃ | 16 | 30 |
| 20 | NHCO | CH₂CH₂ | NH | CH=CH₂ | 6 | 35 |
| 21 | NHCO | CH₂CH₂CH₂ | NCH₃ | CH=CHCH₃ | 4 | 25 |
| 22 | NHCO | CH₂CH₂CH₂CH₂ | O | C≡CH | 4 | 30 |
| 23 | NHCO | CH₂CH₂CH₂CH₂CH₂ | NH | C≡CCH₃ | 4 | 35 |
| 24 | NHCO | CH₂CH₂CH₂CH₂CH₂CH₂ | NCH₃ | nitrile | 4 | 25 |
| 25 | CONH | CH₂ | O | thiol | 14 | 30 |
| 26 | CONH | CH₂CH₂ | NH | maleimide | 4 | 35 |
| 27 | CONH | CH₂CH₂CH₂ | NCH₃ | epoxide | 4 | 25 |
| 28 | CONH | CH₂CH₂CH₂CH₂ | O | aziridine | 4 | 30 |
| 29 | CONH | CH₂CH₂CH₂CH₂CH₂ | NH | thiirane | 4 | 35 |
| 30 | CONH | CH₂CH₂CH₂CH₂CH₂CH₂ | NCH₃ | triazole | 4 | 30 |

In Table 1 above, P represents CH₂CH₂CH₂CH₂C*H (NH₂) (wherein the chiral carbon is an R configuration).

**[Table 2]**

| Compound No. | X | Y¹ | Y² | Z | m | n |
|---|---|---|---|---|---|---|
| 31 | O | CH₂ | O | N3 | 4 | 30 |
| 32 | NH | CH₂CH₂ | NH | N3 | 10 | 80 |
| 33 | NH | CH₂CH₂CH₂ | NCH₃ | N3 | 4 | 90 |
| 34 | NCH₃ | CH₂CH₂CH₂CH₂CH₂CH₂ | O | N3 | 4 | 25 |
| 35 | NHCO | CH₂ | NH | N3 | 16 | 30 |
| 36 | O | CH₂CH₂ | NCH₃ | CH=CH₂ | 6 | 35 |
| 37 | O | CH₂CH₂CH₂ | O | CH=CHCH₃ | 4 | 25 |
| 38 | O | CH₂CH₂CH₂CH₂CH₂ | NH | C≡CH | 4 | 30 |
| 39 | O | CH₂CH₂CH₂CH₂CH₂ | NCH₃ | C≡CCH₃ | 4 | 35 |
| 40 | O | CH₂ | O | nitrile | 4 | 25 |
| 41 | NCH₃ | CH₂CH₂CH₂CH₂CH₂ | NH | thiol | 14 | 30 |
| 42 | NCH₃ | CH₂CH₂ | NCH₃ | maleimide | 4 | 35 |
| 43 | CONH | CH₂CH₂CH₂ | O | epoxide | 4 | 25 |
| 44 | CONH | CH₂CH₂CH₂CH₂ | NH | aziridine | 4 | 30 |
| 45 | CONH | CH₂ | NCH₃ | thiirane | 4 | 35 |
| 46 | CONH | CH₂CH₂CH₂CH₂CH₂CH₂ | O | triazole | 4 | 30 |
| 47 | O | CH₂ | NH | N3 | 4 | 30 |
| 48 | NH | CH₂CH₂ | NCH₃ | N3 | 10 | 80 |
| 49 | NH | CH₂CH₂CH₂ | O | N3 | 4 | 90 |
| 50 | NCH₃ | CH₂CH₂CH₂CH₂CH₂CH₂ | NH | N3 | 4 | 25 |
| 51 | NHCO | CH₂ | NCH₃ | N3 | 16 | 30 |
| 52 | O | CH₂CH₂ | O | CH=CH₂ | 6 | 35 |
| 53 | O | CH₂CH₂CH₂ | NH | CH=CHCH₃ | 4 | 25 |
| 54 | O | CH₂CH₂CH₂CH₂CH₂ | NCH₃ | C≡CH | 4 | 30 |
| 55 | O | CH₂CH₂CH₂CH₂CH₂ | O | C≡CCH₃ | 4 | 35 |
| 56 | O | CH₂ | NH | nitrile | 4 | 25 |
| 57 | NCH₃ | CH₂CH₂CH₂CH₂CH₂ | NCH₃ | thiol | 14 | 30 |
| 58 | NCH₃ | CH₂CH₂ | O | maleimide | 4 | 35 |
| 59 | CONH | CH₂CH₂CH₂ | NH | epoxide | 4 | 25 |
| 60 | CONH | CH₂CH₂CH₂CH₂ | NCH₃ | aziridine | 4 | 30 |

In Table 2 above, P in compound numbers 31 to 45 represents CH₂CH₂CH₂C*H(NH₂)CH₂ (wherein the chiral carbon is an S configuration), and P in compound numbers 46 to 60 represents CH₂CH₂CH₂C*H(NH₂)(wherein the chiral carbon is an R configuration).

**[Table 3]**

| Compound No. | X | Y¹ | Y² | Z | m | n |
|---|---|---|---|---|---|---|
| 61 | O | CH₂ | O | N₃ | 4 | 30 |
| 62 | NH | CH₂CH₂ | NH | N₃ | 10 | 80 |
| 63 | NH | CH₂CH₂CH₂ | NCH₃ | N₃ | 4 | 90 |
| 64 | NCH₃ | CH₂CH₂CH₂CH₂CH₂CH₂ | O | N₃ | 4 | 25 |
| 65 | NHCO | CH₂ | NH | N₃ | 16 | 30 |
| 66 | O | CH₂CH₂ | NCH₃ | CH=CH₂ | 6 | 35 |
| 67 | O | CH₂CH₂CH₂ | O | CH=CHCH₃ | 4 | 25 |
| 68 | O | CH₂CH₂CH₂CH₂CH₂ | NH | C≡CH | 4 | 30 |
| 69 | O | CH₂CH₂CH₂CH₂CH₂ | NCH₃ | C≡CCH₃ | 4 | 35 |
| 70 | O | CH₂ | O | nitrile | 4 | 25 |
| 71 | NCH₃ | CH₂CH₂CH₂CH₂CH₂ | NH | thiol | 14 | 30 |
| 72 | NCH₃ | CH₂CH₂ | NCH₃ | maleimide | 4 | 35 |
| 73 | CONH | CH₂CH₂CH₂ | O | epoxide | 4 | 25 |
| 74 | CONH | CH₂CH₂CH₂CH₂ | NH | aziridine | 4 | 30 |
| 75 | O | CH₂ | NCH₃ | N₃ | 4 | 30 |
| 76 | NH | CH₂CH₂ | O | N₃ | 10 | 80 |
| 77 | NH | CH₂CH₂CH₂ | NH | N₃ | 4 | 90 |
| 78 | NCH₃ | CH₂CH₂CH₂CH₂CH₂CH₂ | NCH₃ | N₃ | 4 | 25 |
| 79 | NHCO | CH₂ | O | N₃ | 16 | 30 |
| 80 | O | CH₂CH₂ | NH | CH=CH₂ | 6 | 35 |
| 81 | O | CH₂CH₂CH₂ | NCH₃ | CH=CHCH₃ | 4 | 25 |
| 82 | O | CH₂CH₂CH₂CH₂CH₂ | O | C≡CH | 4 | 30 |
| 83 | O | CH₂CH₂CH₂CH₂CH₂ | NH | C≡CCH₃ | 4 | 35 |
| 84 | O | CH₂ | NCH₃ | nitrile | 4 | 25 |
| 85 | NCH₃ | CH₂CH₂CH₂CH₂CH₂ | O | thiol | 14 | 30 |
| 86 | NCH₃ | CH₂CH₂ | NH | maleimide | 4 | 35 |
| 87 | CONH | CH₂CH₂CH₂ | NCH₃ | epoxide | 4 | 25 |
| 88 | CONH | CH₂CH₂CH₂CH₂ | O | aziridine | 4 | 30 |
| 89 | CONH | CH₂ | NH | thiirane | 4 | 35 |
| 90 | CONH | CH₂CH₂CH₂CH₂CH₂CH₂ | NCH₃ | triazole | 4 | 30 |

In Table 3 above, P in compound numbers 61 to 70 represents CH₂CH₂C*H(NH₂)CH₂ (wherein the chiral carbon is an S configuration), P in compound numbers 71 to 80 represents CH₂CH₂C*H(NH₂)(wherein the chiral carbon is an R configuration), and P in compound numbers 81 to 90 represents CH₂C*H(NH₂) (wherein the chiral carbon is an S configuration).

Specific examples of the compound represented by the general formula (1) (in the case where Q is a structure represented by the general formula (3)) are shown below.

**[Table 4]**

| Compound No. | X | Y | Z | m | n |
|---|---|---|---|---|---|
| 91 | O | CH₂ | N₃ | 4 | 30 |
| 92 | O | CH₂CH₂ | N₃ | 12 | 10 |
| 93 | O | CH₂CH₂CH₂ | N₃ | 4 | 20 |
| 94 | O | CH₂CH₂CH₂CH₂ | N₃ | 4 | 40 |
| 95 | O | CH₂CH₂CH₂CH₂CH₂ | N₃ | 4 | 50 |
| 96 | O | CH₂CH₂CH₂CH₂CH₂CH₂ | N₃ | 2 | 60 |
| 97 | NH | CH₂ | N₃ | 30 | 70 |
| 98 | NH | CH₂CH₂ | N₃ | 10 | 80 |
| 99 | NH | CH₂CH₂CH₂ | N₃ | 4 | 90 |
| 100 | NH | CH₂CH₂CH₂CH₂ | N₃ | 4 | 100 |
| 101 | NH | CH₂CH₂CH₂CH₂CH₂ | N₃ | 4 | 2 |
| 102 | NH | CH₂CH₂CH₂CH₂CH₂CH₂ | N₃ | 4 | 25 |
| 103 | NCH₃ | CH₂ | N₃ | 20 | 30 |
| 104 | NCH₃ | CH₂CH₂ | N₃ | 8 | 35 |
| 105 | NCH₃ | CH₂CH₂CH₂ | N₃ | 4 | 25 |
| 106 | NCH₃ | CH₂CH₂CH₂CH₂ | N₃ | 4 | 30 |
| 107 | NCH₃ | CH₂CH₂CH₂CH₂CH₂ | N₃ | 4 | 35 |
| 108 | NCH₃ | CH₂CH₂CH₂CH₂CH₂CH₂ | N₃ | 4 | 25 |
| 109 | NHCO | CH₂ | N₃ | 16 | 30 |
| 110 | NHCO | CH₂CH₂ | CH=CH₂ | 6 | 35 |
| 111 | NHCO | CH₂CH₂CH₂ | CH=CHCH₃ | 4 | 25 |
| 112 | NHCO | CH₂CH₂CH₂CH₂ | C≡CH | 4 | 30 |
| 113 | NHCO | CH₂CH₂CH₂CH₂CH₂ | C≡CCH₃ | 4 | 35 |
| 114 | NHCO | CH₂CH₂CH₂CH₂CH₂CH₂ | nitrile | 4 | 25 |
| 115 | CONH | CH₂ | thiol | 14 | 30 |
| 116 | CONH | CH₂CH₂ | maleimide | 4 | 35 |
| 117 | CONH | CH₂CH₂CH₂ | epoxide | 4 | 25 |
| 118 | CONH | CH₂CH₂CH₂CH₂ | aziridine | 4 | 30 |
| 119 | CONH | CH₂CH₂CH₂CH₂CH₂ | thiirane | 4 | 35 |
| 120 | CONH | CH₂CH₂CH₂CH₂CH₂CH₂ | triazole | 4 | 30 |

In Table 4 above, P represents CH₂CH₂CH₂CH₂C*H (NH₂)(wherein the chiral carbon is an S configuration).

**[Table 5]**

| Compound No. | X | Y | Z | m | n |
|---|---|---|---|---|---|
| 121 | O | CH₂ | N₃ | 4 | 30 |
| 122 | NH | CH₂CH₂ | N₃ | 10 | 80 |
| 123 | NH | CH₂CH₂CH₂ | N₃ | 4 | 90 |
| 124 | NCH₃ | CH₂CH₂CH₂CH₂CH₂CH₂ | N₃ | 4 | 25 |
| 125 | NHCO | CH₂ | N₃ | 16 | 30 |
| 126 | O | CH₂CH₂ | CH=CH₂ | 6 | 35 |
| 127 | O | CH₂CH₂CH₂ | CH=CHCH₃ | 4 | 25 |
| 128 | O | CH₂CH₂CH₂CH₂CH₂ | C≡CH | 4 | 30 |
| 129 | O | CH₂CH₂CH₂CH₂CH₂ | C≡CCH₃ | 4 | 35 |
| 130 | O | CH₂ | nitrile | 4 | 25 |
| 131 | NCH₃ | CH₂CH₂CH₂CH₂CH₂ | thiol | 14 | 30 |
| 132 | NCH₃ | CH₂CH₂ | maleimide | 4 | 35 |
| 133 | CONH | CH₂CH₂CH₂ | epoxide | 4 | 25 |
| 134 | CONH | CH₂CH₂CH₂CH₂ | aziridine | 4 | 30 |
| 135 | CONH | CH₂ | thiirane | 4 | 35 |
| 136 | CONH | CH₂CH₂CH₂CH₂CH₂CH₂ | triazole | 4 | 30 |
| 137 | O | CH₂ | N₃ | 4 | 30 |
| 138 | NH | CH₂CH₂ | N₃ | 10 | 80 |
| 139 | NH | CH₂CH₂CH₂ | N₃ | 4 | 90 |
| 140 | NCH₃ | CH₂CH₂CH₂CH₂CH₂CH₂ | N₃ | 4 | 25 |
| 141 | NHCO | CH₂ | N₃ | 16 | 30 |
| 142 | O | CH₂CH₂ | CH=CH₂ | 6 | 35 |
| 143 | O | CH₂CH₂CH₂ | CH=CHCH₃ | 4 | 25 |
| 144 | O | CH₂CH₂CH₂CH₂CH₂ | C≡CH | 4 | 30 |
| 145 | O | CH₂CH₂CH₂CH₂CH₂ | C≡CCH₃ | 4 | 35 |
| 146 | O | CH₂ | nitrile | 4 | 25 |
| 147 | NCH₃ | CH₂CH₂CH₂CH₂CH₂ | thiol | 14 | 30 |
| 148 | NCH₃ | CH₂CH₂ | maleimide | 4 | 35 |
| 149 | CONH | CH₂CH₂CH₂ | epoxide | 4 | 25 |
| 150 | CONH | CH₂CH₂CH₂CH₂ | aziridine | 4 | 30 |

In Table 5 above, P in compound numbers 121 to 135 represents CH₂CH₂CH₂C*H(NH₂)CH₂ (wherein the chiral carbon is an R configuration), and P in compound numbers 136 to 150 represents CH₂CH₂CH₂C*H(NH₂)(wherein the chiral carbon is an S configuration).

**[Table 6]**

| Compound No. | X | Y | Z | m | n |
|---|---|---|---|---|---|
| 151 | O | CH₂ | N₃ | 4 | 30 |
| 152 | NH | CH₂CH₂ | N₃ | 10 | 80 |
| 153 | NH | CH₂CH₂CH₂ | N₃ | 4 | 90 |
| 154 | NCH₃ | CH₂CH₂CH₂CH₂CH₂CH₂ | N₃ | 4 | 25 |
| 155 | NHCO | CH₂ | N₃ | 16 | 30 |
| 156 | O | CH₂CH₂ | CH=CH₂ | 6 | 35 |
| 157 | O | CH₂CH₂CH₂ | CH=CHCH₃ | 4 | 25 |
| 158 | O | CH₂CH₂CH₂CH₂CH₂ | C≡CH | 4 | 30 |
| 159 | O | CH₂CH₂CH₂CH₂CH₂ | C≡CCH₃ | 4 | 35 |
| 160 | O | CH₂ | nitrile | 4 | 25 |
| 161 | NCH₃ | CH₂CH₂CH₂CH₂CH₂ | thiol | 14 | 30 |
| 162 | NCH₃ | CH₂CH₂ | maleimide | 4 | 35 |
| 163 | CONH | CH₂CH₂CH₂ | epoxide | 4 | 25 |
| 164 | CONH | CH₂CH₂CH₂CH₂ | aziridine | 4 | 30 |
| 165 | O | CH₂ | N3 | 4 | 30 |
| 166 | NH | CH₂CH₂ | N3 | 10 | 80 |
| 167 | NH | CH₂CH₂CH₂ | N3 | 4 | 90 |
| 168 | NCH₃ | CH₂CH₂CH₂CH₂CH₂CH₂ | N3 | 4 | 25 |
| 169 | NHCO | CH₂ | N3 | 16 | 30 |
| 170 | O | CH₂CH₂ | CH=CH₂ | 6 | 35 |
| 171 | O | CH₂CH₂CH₂ | CH=CHCH₃ | 4 | 25 |
| 172 | O | CH₂CH₂CH₂CH₂CH₂ | C≡CH | 4 | 30 |
| 173 | O | CH₂CH₂CH₂CH₂CH₂ | C≡CCH₃ | 4 | 35 |
| 174 | O | CH₂ | nitrile | 4 | 25 |
| 175 | NCH₃ | CH₂CH₂CH₂CH₂CH₂ | thiol | 14 | 30 |
| 176 | NCH₃ | CH₂CH₂ | maleimide | 4 | 35 |
| 177 | CONH | CH₂CH₂CH₂ | epoxide | 4 | 25 |
| 178 | CONH | CH₂CH₂CH₂CH₂ | aziridine | 4 | 30 |
| 179 | CONH | CH₂ | thiirane | 4 | 35 |
| 180 | CONH | CH₂CH₂CH₂CH₂CH₂CH₂ | triazole | 4 | 30 |

In Table 6 above, P in compound numbers 151 to 160 represents CH₂CH₂C^{∗}H(NH₂)CH₂ (wherein the chiral carbon is an R configuration), P in compound numbers 161 to 170 represents CH₂CH₂C^{∗}H(NH₂) (wherein the chiral carbon is an S configuration), and P in compound numbers 171 to 180 represents CH₂C^{∗}H(NH₂) (wherein the chiral carbon is an R configuration).

Examples of the pharmacologically acceptable salt of the compound represented by the general formula (1) or (4) include salts with inorganic acids such as hydrochloric acid, sulfuric acid, and phosphoric acid; salts with organic acids such as oxalic acid, maleic acid, succinic acid, and acetic acid; salts with inorganic bases such as sodium and potassium; and salts with organic bases such as dimethylamine and triethylamine.

The compound represented by the general formula (1) or a salt thereof can be produced by condensation of a compound represented by the general formula (6): 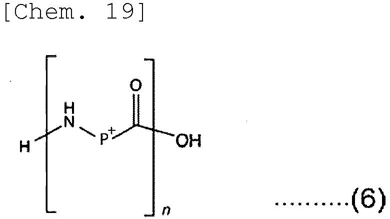 (wherein P⁺ represents a (C₁-C₅) alkylene group substituted by one or more (preferably one) N⁺H₃ groups, and n is as defined above) and
a compound represented by the general formula HQZ (wherein Q and Z are as defined above) or a salt thereof.

The compound represented by the general formula (4) or a salt thereof can be produced by condensation of an ε-PL represented by the general formula (7): 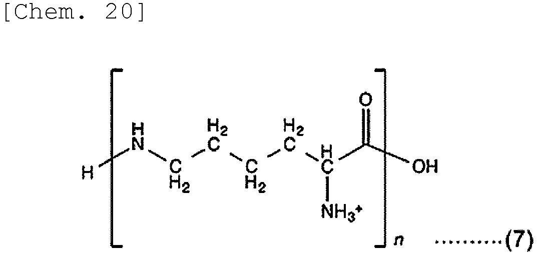 (wherein n is as defined above) and
a compound represented by the general formula (3a): 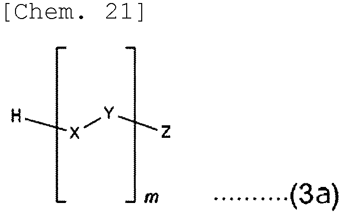 (wherein X, Y, and m are as defined above) or a salt thereof.

The amino group of the starting material which is not supposed to participate in the condensation reaction is protected by a protective group before the reaction and deprotected after the reaction. Thus, the desired compound, i.e., the compound represented by the general formula (1) or a salt thereof can be produced. The condensation procedure is well established in this field and can be performed in a well-established manner in the present invention. The post-reaction mixture containing the desired compound is subjected to usual treatments such as concentration, phase transfer, and chromatography to give the compound represented by the general formula (1). Such techniques are well established in the field of peptide synthesis and can be performed in a well-established manner in the present invention.

The compound represented by the general formula (6) (including ε-PL, i.e., the compound represented by the general formula (7)) may be produced by fermentation or a chemical reaction such as chemical synthesis, or may be chemically synthesized from lysine. For example, the strain of *Streptomyces albulus* subsp. *lysinopolymerus* described in Japanese Patent No. 1245361 is cultured in a medium, for example, a medium which contains 5 wt% glucose, 0.5 wt% yeast extract, 1 wt% ammonium sulfate, 0.08 wt% dipotassium hydrogen phosphate, 0.136 wt% potassium dihydrogen phosphate, 0.05 wt% magnesium sulfate heptahydrate, 0.004 wt% zinc sulfate heptahydrate, and 0.03 wt% iron sulfate heptahydrate and is adjusted to pH 6.8, and from the resulting culture, ε-PL can be isolated and harvested.

The polycationic compounds including those similar to the ε-PL represented by the general formula (7) may be obtained by any appropriate method, and for example, can be obtained by the methods described in the following Non Patent Literature.

For example, γ-poly-L-diaminobutanoic acid can be produced, for example, as described in the literature (Takehara, M., Saimura, M., Inaba, H. & Hirohara, H. Poly (y-L-diaminobutanoic acid), a novel poly(amino acid), coproduced with poly (ε-L-lysine) by two strains of Streptomyces celluloflavus. FEMS Microbiol Lett 286, 110-7 (2008)).

γ-poly-D-diaminobutanoic acid can be produced, for example, as described in the literature (Ohkuma, H., Tenmyo, O., Konishi, M., Oki, T. & Kawaguchi, H. BMY-28190, a novel antiviral antibiotic complex. J Antibiot (Tokyo) 41, 849-54 (1988)).

β-poly-L-diaminopropionic acid can be produced, for example, as described in the literature (Xia, J., Xu, H., Feng, X., Xu, Z. & Chi, B. Poly(L-diaminopropionic acid), a novel non-proteinic amino acid oligomer co-produced with poly (ε-L-lysine) by Streptomyces albulus PD-1. Appl Microbiol Biotechnol 97, 7597-605 (2013), or Xu, Z. et al., Systematic unravelling of the biosynthesis of poly(L-diaminopropionic acid) in Streptomyces albulus PD-1. Sci Rep 5, 17400 (2015)).

In the present invention, the polycationic compound such as the ε-PL represented by the general formula (7) may be used as a salt. Examples of the salt include inorganic acid salts of the polycationic compound formed with inorganic acids such as hydrochloric acid, hydrobromic acid, sulfuric acid, and phosphoric acid; organic acid salts of the polycationic compound formed with organic acids such as acetic acid, formic acid, propionic acid, fumaric acid, malic acid, citric acid, maleic acid, adipic acid, gluconic acid, and lactic acid; saturated fatty acid salts of the polycationic compound formed with medium- and long-chain saturated fatty acids such as caproic acid, lauric acid, and stearic acid; and unsaturated fatty acid salts of the polycationic compound formed with medium- and long-chain unsaturated fatty acids such as oleic acid, linoleic acid, and arachidonic acid.

The compound represented by the general formula (2a) or (3a) is a known compound or a compound similar to the known compound. Therefore, the compound represented by the general formula (2a) or (3a) can be easily produced according to a known method or a method known per se.

The compound represented by the general formula (1) or (4) can be produced in a simple and efficient manner by the method based on microbial culture as described later.

The microbe used in the present invention may be any microbe capable of producing a polycationic compound such as ε-PL, but is preferably a *Kitasatospora* or *Streptomyces* bacterium.

Examples of the *Kitasatospora* bacterium include those described in Interenational Journal of Systematic Bacteriology, vol. 47, pp. 1048-1054, 1997. Among them, *Kitasatospora kifunense* is preferable, and *Kitasatospora kifunense* strain MN-1 (FERM P-19712) is more preferable.

Examples of the *Streptomyces* bacterium include *Streptomyces albulus* subsp. *lysinopolymerus* strain No. 346-D (IFO14147, JP-B 59-20359), *Streptomyces albulus* subsp. *lysinopolymerus* strain 11011A (JP-B 3-42070), *Streptomyces albulus* subsp. *lysinopolymerus* strain B21021 (JP-A 09-173057), *Streptomyces albulus* subsp. *lysinopolymerus* strain B15208 (JP-A 10-290688), *Streptomyces albulus* subsp. strain SP-25 (JP-A 2002-095467), *Streptomyces* sp. strain SP-72 (JP-A 2000-069988), *Streptomyces* sp. strain SP-66 (JP-A 2001-017159), *Streptomyces herbaricolor* strain SP-13 (JP-A 2002-095466), *Streptomyces lavendulae* strain USE-53 (JP-A 2003-052358), and *Streptomyces noursei* (JP-A 1-187090) . Among them, the members of *Streptomyces albulus* subsp. are preferable, and *Streptomyces albulus* subsp. *lysinopolymerus* strain No. 346-D is more preferable (identical to *Streptomyces albulus* strain NBRC14147).

The microbe used in the present invention may also be a microbe capable of producing the above-mentioned compounds similar to ε-PL, such as γ-poly-L-diaminobutanoic acid, γ-poly-D-diaminobutanoic acid, and β-poly-L-diaminopropionic acid.

The medium for culturing these microbes is supplemented with the compound represented by the general formula (2a) or (3a), which is a compound containing a click functional group. The compound represented by the general formula (2a) or (3a) may be any compound that is encompassed in the scope of the compound represented by the general formula (2a) or (3a). Examples include PEG-azide and PEG-alkyne (that is, alkynyl PEG). The concentration of the compound represented by the general formula (2a) or (3a) contained in the medium is preferably 0.1% to 1.0% (% refers to percentage by volume). The medium to be supplemented with the compound represented by the general formula (2a) or (3a) may be any medium containing a carbon source, a nitrogen source, an inorganic substance, other nutrients, etc. as appropriate. The carbon source may be any carbon source that the polycationic compound-producing strain can assimilate, and examples include glucose, fructose, starch, and glycerol. The amount of the carbon source in the medium is preferably 1.0% to 10.0%, and more preferably 1.0% to 5.0%. Examples of the nitrogen source include peptone, casein hydrolysate, amino acid, inorganic ammonium salt, and ammonium sulfate, and particularly preferred is ammonium sulfate. The amount of the nitrogen source in the medium is preferably 0.1% to 5.0%, and more preferably 0.1% to 1.0%. The carbon source or the nitrogen source may be sequentially added to the medium during the culture. Examples of the inorganic substance include phosphate ion, potassium ion, sodium ion, magnesium ion, zinc ion, iron ion, manganese ion, nickel ion, and sulfate ion. The medium preferably contains yeast extract to facilitate microbial growth. The amount of the yeast extract in the medium is preferably 0.05% to 0.5%, and more preferably 0.1% to 0.5%. The pH of the medium may be any value suitable for culturing microbes, for example pH 3 to 8. In particular, near-neutral pH (pH 6 to 8) is preferable for microbial growth, and pH 3 to 5 is more preferable for the production of the compound represented by the general formula (1). In the present invention, % refers to w/v % unless otherwise specified. For more details, see the EXAMPLES shown below.

The culture is preferably performed with agitation, stirring, or the like under aerobic conditions while oxygen is being supplied. The culture temperature is not particularly limited and may be any temperature suitable for the growth of the polycationic compound-producing strain, for example 25 to 35°C. After the culture starts, the pH of the culture broth decreases as microbes grow. For example, by adding an alkaline solution to the culture broth, the pH of the culture broth can be maintained at around 4. The alkaline solution to be added to the culture broth is not particularly limited and may be, for example, sodium hydroxide, aqueous ammonia solution, or the like.

After removing microbial cells from the culture broth by centrifugation or filter filtration, the microbial cell-free fluid is passed through a weakly acidic cation exchange resin so that the compound of interest is collected by adsorption onto the resin. The weakly acidic cation exchange resin is not particularly limited and may be, for example, Amberlite IRC-50 (manufactured by Rohm and Haas Company Japan) or the like. After adsorption, elution with hydrochloric acid gives the polycationic compound derivative represented by the general formula (1) in high purity. Alternatively, the polycationic compound derivative can be isolated and purified by the tetraphenylborate method as described in the literature (Non Patent Literature, H. Katano et al., Anal. Sci., 28, 1153-1157, 2012) .

Subsequently, the compound represented by the general formula (1) is attached to a pharmacological substance by a covalent bond by click chemistry. The click chemistry is well known to the skilled person. The click chemistry can be performed in a well-known manner in the present invention. The pharmacological substance used in the present invention may be any pharmacological substance that can be conjugated to the compound represented by the general formula (1) or a salt thereof by covalently bonding to the click functional group. For example, a compound having a triple bond (e.g., -C≡C-) is preferred. In short, preferred are pharmacological substances that react with the click functional group and form a chemical bond therewith. This attachment improves the biomembrane permeability as well as water solubility of the pharmacological substance.

Examples of the pharmacological substance include antimicrobial agents including antifungal agents, antibacterial agents, anticancer agents, proteins such as enzymes and antibodies, and genes. Examples of the antifungal agent include polyene antibiotics (amphotericin B, nystatin, trichomycin, pimaricin, etc.), micafungin, itraconazole, ketoconazole, fluconazole, miconazole, and flucytosine. Examples of the antibacterial agent include β-lactam antibiotics (penicillin, ampicillin, talampicillin, bacampicillin, cephalosporin C, cefalexin, cefradine, cephamycin, imipenem, etc.), aminoglycoside antibiotics (streptomycin, gentamicin, kanamycin, tobramycin, amikacin, arbekacin, ribostamycin, etc.), tetracycline antibiotics (oxytetracycline, tetracycline, etc.), and peptide antibiotics (bacitracin, gramicidin, polymyxin B, vancomycin, teicoplanin, etc.). Examples of the anticancer agent include anticancer agents that can form a bond with the click functional group. Examples of the anticancer agent that can form a bond with the click functional group include actinomycin D, mitomycin, anthracycline antineoplastic agents (doxorubicin, daunorubicin, aclarubicin, etc.), bleomycin, and cisplatin. Examples of the protein include fluorescent proteins such as mAG; Cas proteins used for CRISPR-Cas9 system (Cas, Cas9); Yamanaka factor proteins, which are protein products of the genes such as Oct3/4, Sox2, Klf4, and c-Myc; and gene expression regulatory proteins. Examples of the enzyme include trypsin, Cre recombinase, and λ-red recombinases. Examples of the antibody include immunoglobulins such as an IgG antibody, an IgA antibody, and an IgY antibody. Examples of the gene include plasmids; oligonucleotides; Yamanaka factors such as Oct3/4, Sox2, Klf4, and c-Myc; and genes used for CRISPR-Cas9 system, such as PAM sequence and gRNA (guide RNA, guide RNA, sgRNA).

For attaching the compound represented by the general formula (1) or a salt thereof to the pharmacological substance, it is convenient to use a conventional intervening group used in the field of click chemistry. In short, the intervening group is a group which intervenes to connect the pharmacological substance to the compound represented by the general formula (1) or a salt thereof. The intervening group may be any group that does not interfere with the object of the present invention. A more specific description is made with reference to, for example, the compounds represented by the formulae (9a) to (9d) shown below, which serve as the base of the intervening group. The amino group, the carboxyl group, or the hydroxyl group in the compounds is reacted with a carboxyl group, a hydroxyl group, or an amino group in a pharmacological substance to form a covalent bond. The triple bond in the compounds is then reacted to the click functional group in the compound represented by the general formula (1) or a salt thereof to form a covalent bond, resulting in connection of the pharmacological substance with the compound represented by the general formula (1) or a salt thereof. Such a chemical entity for connecting the pharmacological substance with the compound represented by the general formula (1) or a salt thereof is an example of the intervening group. The type of the intervening group and the procedure for attaching the intervening group to the pharmacological substance (amidation, esterification, Huisgen reaction, etc.), etc. are well established in the field of click chemistry and can be selected from well-established ones as appropriate in the present invention.

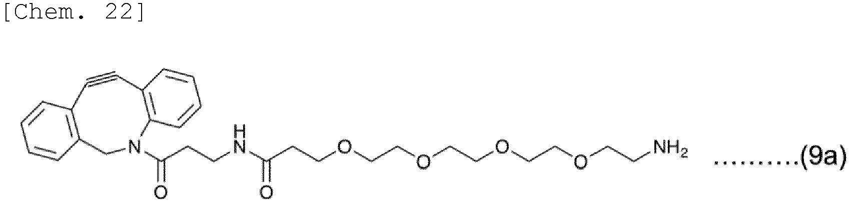

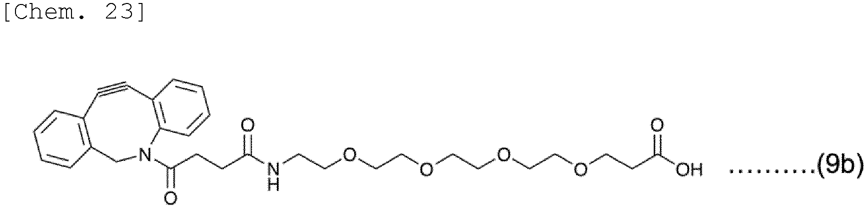

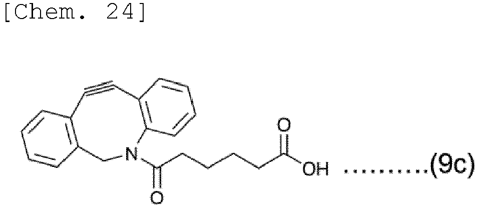

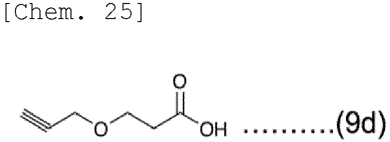

For attaching the compound represented by the general formula (1) or a salt thereof to the pharmacological substance, an intervening compound for click chemistry, such as DBCO (dibenzocyclooctyne) may be used. That is, it is advantageous to attach the compound represented by the general formula (1) or a salt thereof to a compound obtained by conjugating the pharmacological substance to an intervening compound for click chemistry, such as DBCO.

Examples are given below to illustrate the attachment of the compound represented by the general formula (1) to the pharmacological substance by a covalent bond.

From a poorly water-soluble Amp-B represented by formula (8) : 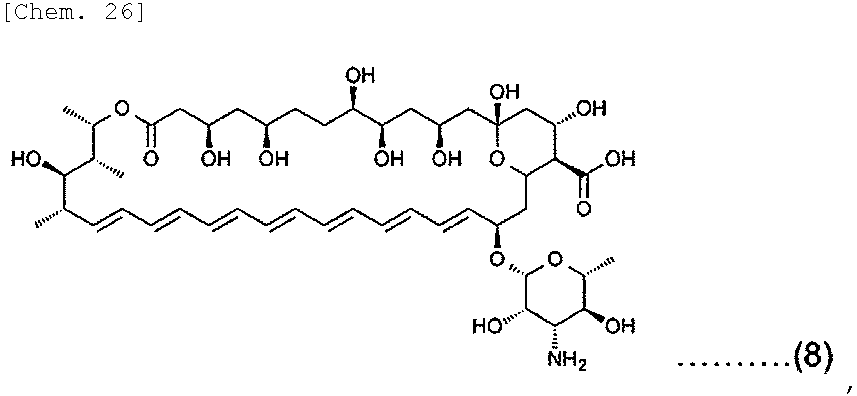 AmpB-DBCO represented by formula (9): 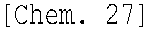 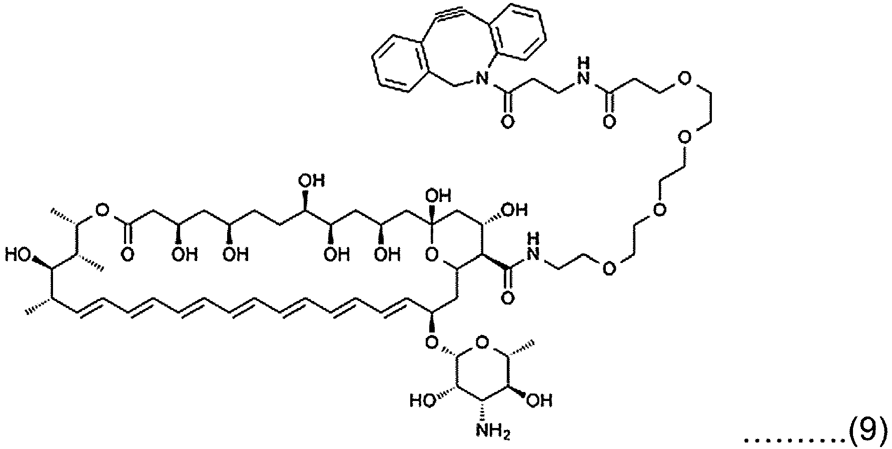 is produced by organic synthesis. This reaction may be performed by a known method or an amidation reaction known per se. The thus-produced compound (9) or a salt thereof is reacted with an ε-PL derivative (hereinafter referred to as ε-PL-PEG-azide) represented the general formula (10): 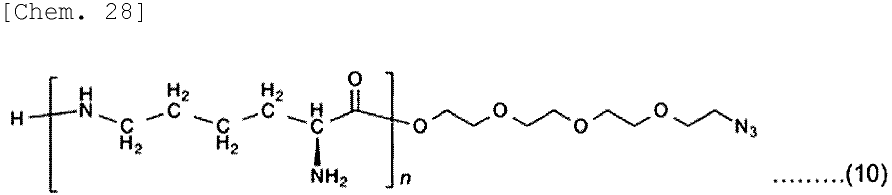 (wherein n is an integer of 2 to 100) or
a salt thereof to yield a water-soluble ε-PL-DBCO-AmpB represented by the general formula (5): 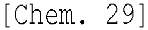 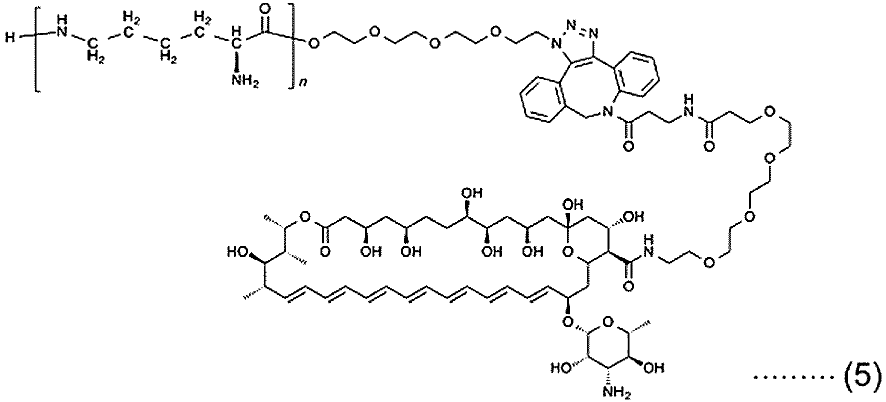 (wherein n is an integer of 2 to 100) or
a salt thereof. For more details, see the EXAMPLES shown below.

The thus-produced water-soluble ε-PL-DBCO-AmpB represented by the general formula (5) or a salt thereof not only retains the antimicrobial activity of amphotericin B, which is useful as a therapeutic agent for deep mycosis, but also has overcome the drawbacks of amphotericin B, such as poor intestinal absorption, which makes oral administration of amphotericin B impractical and limits the choice to intravenous administration, and poor water solubility, which prolongs the infusion time.

A water-soluble ε-PL-DBCO-Dox represented by the general formula (5-2) or a salt thereof can be produced in the same manner as for the compound represented by the general formula (5). For more details, see the EXAMPLES shown below.

The thus-produced water-soluble ε-PL-DBCO-Dox represented by the general formula (5-2) or a salt thereof not only retains the anticancer activity of the useful anticancer agent doxorubicin, but also has an improved intestinal absorption and an improved adverse effect profile. In addition, the Dox moiety of the ε-PL-DBCO-Dox can intercalate the double helix structure of DNA, and therefore, only mixing of the ε-PL-DBCO-Dox with gene fragments, plasmid DNAs, or RNAs enables polycationic modification of DNAs or RNAs without covalent bonds. The polycation-modified plasmid DNAs, gene fragments, or RNAs prepared by the method of the present invention can be directly delivered into cells . For more details, see the EXAMPLES shown below.

An ε-PL-DBCO-mAG represented by the general formula (5-3) or a salt thereof can be produced in the same manner as for the compound represented by the general formula (5). For more details, see the EXAMPLES shown below.

The thus-produced ε-PL-DBCO-mAG represented by the general formula (5-3) or a salt thereof enables direct intracellular delivery of the fluorescent protein mAG. Similarly, high-molecular pharmacological substances such as proteins (including enzymes and antibodies) can also be directly delivered into cells after ε-PL modification.

The method of the present invention for attaching a polycationic compound via a click functional group can be applied to, for example, industrial materials (e.g., medical devices or functional materials made of plastics, textiles, etc.) etc. Examples of the medical device include cell culture dishes, medical instruments, and regenerative medical materials. Examples of the functional material include electronic substrates and specially coated glass. The method of the present invention for attaching a polycationic compound can provide industrial materials etc. with, for example, antimicrobial activity, cultured cell adhesive function, hydrophilicity, drip proofness, antistatic function, etc.

Hereinafter, embodiments of the present invention will be described in more detail in the examples shown below. The present invention is not limited to the examples shown below, and it is to be understood that the details can be embodied in various ways. The present invention is not limited to technical embodiments herein, and various modifications can be made within the scope of the appended claims. Embodiments obtainable by suitably combining technical means disclosed herein are also included in the technical scope of the present invention.

### EXAMPLES

### Example 1

### Method for production of compounds represented by general formula (1) by microbes

*Streptomyces albulus* NBRC14147 was used as an ε-PL-producing microbe, and pls L883P/pLAE009/*S*. *albulus* CRM003 (Non Patent Literature: Y. Hamano et al., Appl. Environ. Microbiol., 80, 4993-5000, 2014) was used as a short-chain ε-PL-producing microbe. The media used for culturing these microbes were a sterilized SLB (prepared by dissolving 10.3 g of sucrose; 10 g of tryptone; and 5 g of yeast extract in 1 L of distilled water), and a sterilized M3G medium (prepared by dissolving 50 g of glucose; 10 g of ammonium sulfate; 5 g of yeast extract; 1.36 g of potassium dihydrogen phosphate; 1.58 g of disodium hydrogen phosphate dodecahydrate; 0.04 g of zinc sulfate heptahydrate; 0.03 g of iron(II) sulfate heptahydrate; and 0.5 g of magnesium sulfate heptahydrate in 1 L of distilled water, followed by adjusting the pH to 7.0 with aqueous sodium hydroxide solution).

A spore suspension of *Streptomyces albulus* NBRC14147 or pls L883P/pLAE009/*S*. *albulus* CRM003 was inoculated into 3 mL of the SLB medium, and agitated culture was performed at 28°C for 36 hours. For the culture of pls L883P/pLAE009/*S*. *albulus* CRM003, the antibiotic neomycin was used as a selection drug and added at a concentration of 50 µg/mL to the medium. 500 µL of the culture broth was added to 50 mL of the M3G medium, and culture was continued at 28°C for 8 hours. To the culture broth, 2-[2-[2-(2-propyn-1-yloxy)ethoxy]ethoxy]ethanol (hereinafter referred to as PEG-alkyne) or 2-[2-[2-(2-azidoethoxy)ethoxy]ethoxy]ethanol (hereinafter referred to as PEG-azide), either of which is an exemplary compound represented by the general formula (2a) or (3a), was added at 0.2% (w/v), and agitated culture was further performed at 28°C for 2 to 4 days. The culture broth was centrifuged, and the culture supernatant was obtained.

### Determination of structure of general formula (1)

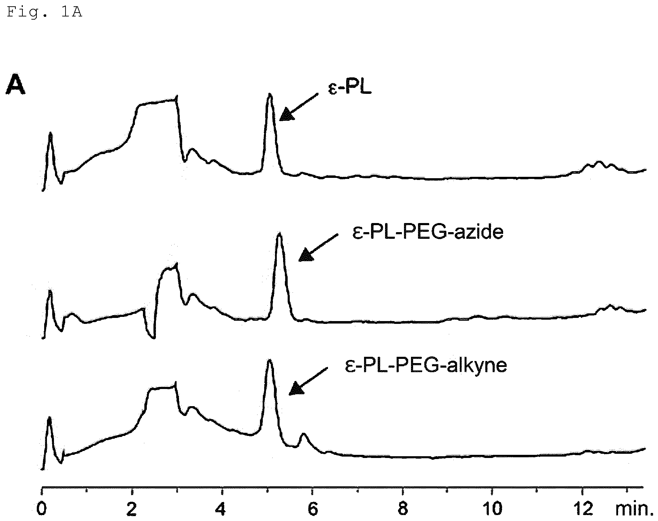

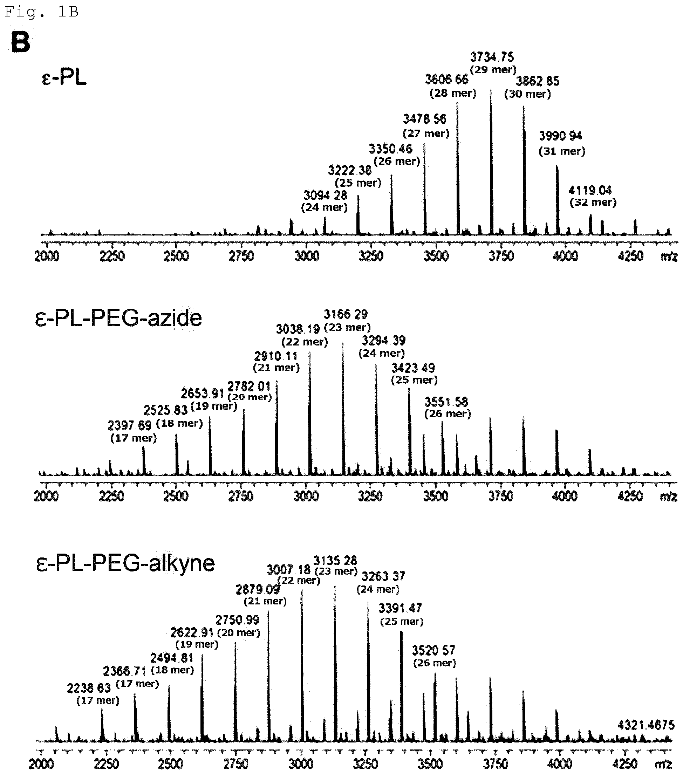

The culture supernatant of *Streptomyces albulus* NBRC14147 obtained in Example 1 was analyzed by electrospray ionization time-of-flight mass spectrometry/high performance liquid chromatography (hereinafter referred to as ESI-TOF-MS/HPLC) for precise molecular weight determination (Figs. 1A and 1B) . The ESI-TOF-MS system used for the ESI-TOF-MS/HPLC analysis was maX is plus manufactured by Bruker Daltonics, and the HPLC system used was Agilent 1290 Infinity LC system manufactured by Agilent Technologies. The HPLC conditions were as follows. The column was SunShell RP-AQUA (manufactured by ChromaNik Technologies Inc., 2.6 µm, inner diameter: 2.1 mm, length: 50 mm) . The column temperature was 40°C. The mobile phase A was 0.05% formic acid and 0.05% heptafluorobutyric acid in water. The mobile phase B was 0.05% formic acid and 0.05% heptafluorobutyric acid in acetonitrile. The flow rate of the mobile phase was 0.3 mL/min. The elution was performed with a linear gradient of 10% to 25% mobile phase B for the initial 3 minutes; a linear gradient of 25% to 90% mobile phase B for a period of 3 to 12 minutes after the start; a linear gradient of 90% to 100% mobile phase B for a period of 12 to 16 minutes after the start; and 100% mobile phase B for a period of 16 to 18 minutes after the start. The eluate was monitored by ESI-TOF-MS.

### Results

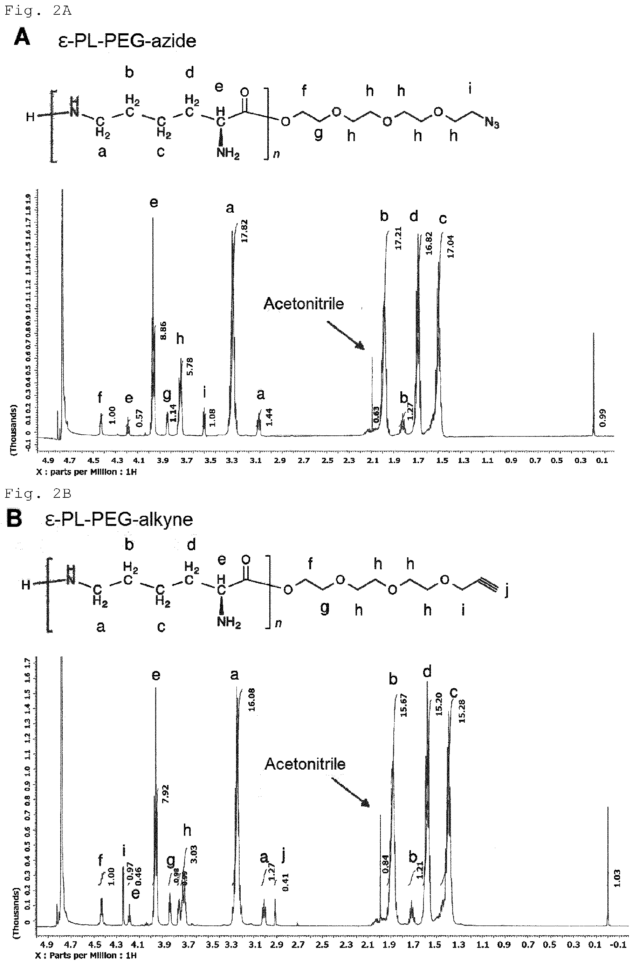

The results of the analysis confirmed the production of an exemplary compound represented by the general formula (1) in which the carboxyl group of ε-PL was conjugated to PEG-azide by an ester bond, i.e., an ε-PL-PEG-azide represented by the general formula (10) (wherein n is an integer of 17 to 26), and the production of an exemplary compound represented by the general formula (1) in which the carboxyl group of ε-PL was conjugated to PEG-alkyne by an ester bond, i.e., an ε-PL-PEG-alkyne represented by the general formula (11) (wherein n is an integer of 17 to 26). Further, the ε-PL-PEG-azide and the ε-PL-PEG-alkyne were isolated from the culture broth and purified by the tetraphenylborate method (Non Patent Literature, H. Katano et al., Anal. Sci., 28, 1153-1157, 2012), and the structure of each compound was determined by nuclear magnetic resonance spectroscopy (¹H-NMR) (Figs. 2A and 2B). The results show that the 17- to 26-mer ε-PL-PEG-azide and the 17- to 26-mer ε-PL-PEG-alkyne can be produced using the ε-PL producing microbe *Streptomyces albulus* NBRC14147. Similarly, a short-chain ε-PL-PEG-azide and a short-chain ε-PL-PEG-alkyne can be produced using the short-chain ε-PL-producing microbe pls L883P/pLAE009/*S*. *albulus* CRM003.

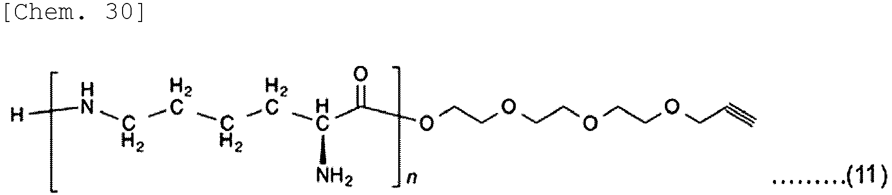

### Example 2

### Improvement of water solubility as well as biomembrane permeability of a compound by ε-PL modification

In order to examine whether ε-PL modification could improve the water solubility as well as biomembrane permeability of a compound, ε-PL modification of a poorly water-soluble fluorescent dye represented by formula (12) (DBCO-PEG₄-5/6-FAM) was performed as a model experiment.

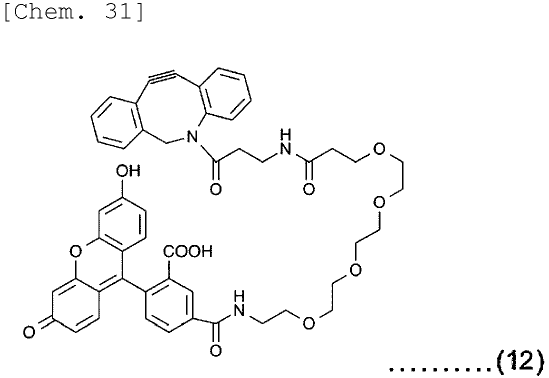

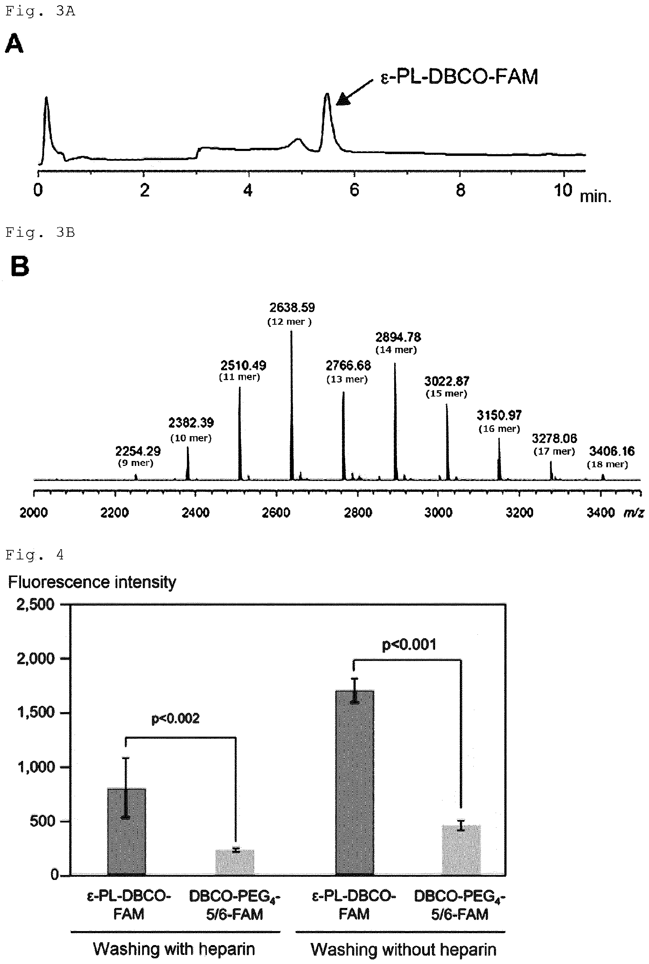

A commercial DBCO-PEG₄-5/6-FAM represented by formula (12) (0.5 µmol) and a 9- to 18-mer ε-PL-PEG-azide represented by the general formula (10) (wherein n is an integer of 9 to 18) (1.0 µmol) were dissolved in 80% dimethylsulfoxide (hereinafter referred to as DMSO). The mixture was subjected to a click chemistry reaction while stirred at 30°C for 24 hours. The reaction mixture was analyzed by ESI-TOF-MS/HPLC as described in Example 1. The results of the analysis confirmed that the entire amount of the DBCO-PEG₄-5/6-FAM added was converted to ε-PL-DBCO-FAM, a compound represented by the general formula (13) (wherein n is an integer of 9 to 18) (Figs. 3A and 3B). The ε-PL-DBCO-FAM purified from the reaction mixture easily dissolved in water, demonstrating that ε-PL modification alters the poorly water-soluble compound DBCO-PEG₄-5/6-FAM to a water-soluble form thereof.

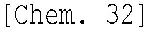

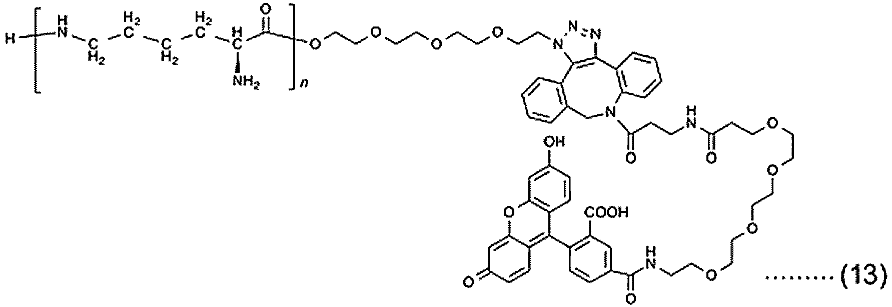

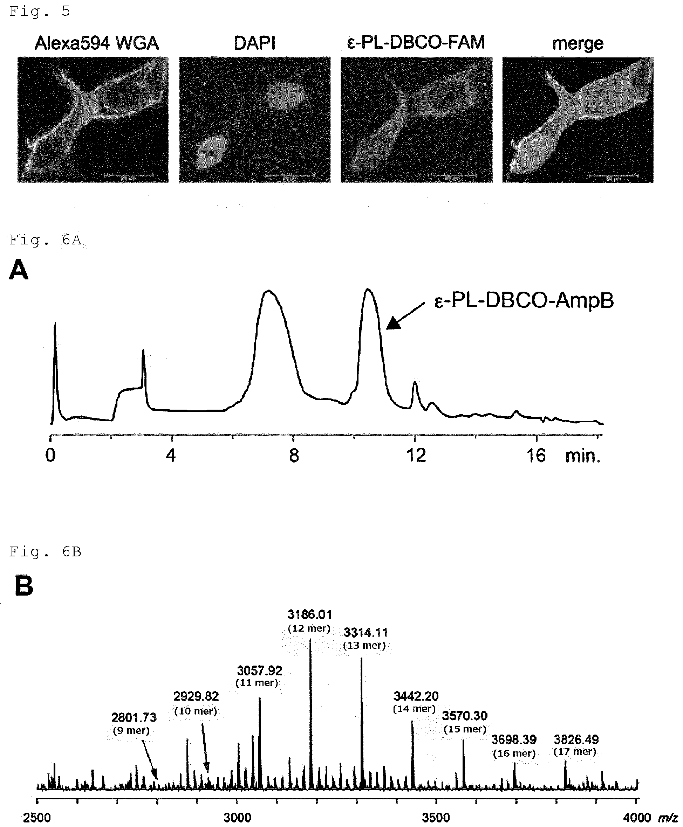

The DBCO-PEG₄-5/6-FAM represented by formula (12) has no biomembrane permeability. An experiment was performed to examine the biomembrane permeability of an ε-PL-modified DBCO-PEG₄-5/6-FAM, that is, the ε-PL-DBCO-FAM represented by the general formula (13) (wherein n is an integer of 9 to 18) using HeLa cells, which are animal cells. The culture of HeLa cells was performed in a Dulbecco's modified Eagle's medium (DMEM) containing 10% fetal bovine serum (FBS) in an atmosphere of 5% carbon dioxide at 37°C. Under such culture conditions, the cells were cultured in 12-well plates until grown to 70 to 80% confluency. The cells were washed twice with DMEM without FBS (DMEM w/o FBS). DMEM w/o FBS was added to the cells, and after cooling to 4°C, the DBCO-PEG₄-5/6-FAM represented by formula (12) or the ε-PL-DBCO-FAM represented by the general formula (13) (wherein n is an integer of 9 to 18) was added at 30 µM. Incubation was performed at 4°C for 30 minutes. The cells were washed successively with phosphate buffered saline (PBS) twice, with PBS alone or PBS containing heparin (100 µg/mL), and with PBS twice. To the cells, 300 µL of 100 mM Tris-HCl buffer solution (pH 8.0) containing 1% Triton X-100 was added for extraction of the compound taken up by the cells. After removing the insoluble fraction by centrifugation, fluorescence intensity was measured. As a result, in both cases where heparin was used and not used to wash the ε-PL-DBCO-FAM bound on the cell surface, the ε-PL-DBCO-FAM represented by the general formula (13) had an about 3.5 times higher biomembrane permeability than that of the DBCO-PEG₄-5/6-FAM represented by formula (12) (Fig. 4). When heparin was not used in washing, the ε-PL-DBCO-FAM-treated cells showed a higher fluorescence intensity, indicating that some of the ε-PL-DBCO-FAM molecules bound to the cell surface were taken up into the cells (Fig. 4). By confocal laser scanning microscopy, the ε-PL-DBCO-FAM represented by the general formula (13) was visually observed to be present in the cells, partially in the nuclei (Fig. 5).

This experiment revealed that ε-PL modification is a solution to improve the water solubility as well as biomembrane permeability of a compound.

### Example 3

ε-PL modification of the antifungal antibiotic AmpB represented by formula (8) was performed to further evaluate the usefulness of the ε-PL-PEG-azide which is an exemplary compound represented by the general formula (1) and is represented by the general formula (10) (wherein n is an integer of 9 to 18). AmpB, which has potent antimicrobial activity against Fungi, is an important antibiotic used in a clinical setting as a therapeutic agent for various fungal infections including deep mycosis. However, AmpB has poor water solubility (is practically insoluble in water), which causes problems such as complicated formulation preparation and adverse effects including nephrotoxicity. Various formulation technologies, such as liposomal formulation, for improving such poor water solubility have currently been studied, but have not yet produced sufficiently satisfactory outcomes. AmpB has poor intestinal absorption in oral administration, and this intestinal absorption problem has not yet been overcome by current formulation technologies. In another context, if an ε-PL modified AmpB could retain potent antifungal activity, the development of a readily water-soluble AmpB could be achieved as well. The present inventors believed that ε-PL modification could solve the set of problems described above.

### ε-PL modification of antifungal antibiotic AmpB

100 mg of AmpB (manufactured by Nacalai Tesque, Inc.) and 73 mg of N-[(9H-fluoren-9-ylmethoxy)carbonyloxy]succinimide (Fmoc-OSu) were dissolved in 30 mL of anhydrous dimethylformamide. The mixture was stirred at 28°C for 4 hours to yield a compound in which the amino group of the amino sugar moiety of AmpB was protected by an Fmoc group (AmpB-Fmoc) . 420 mL of a 25 mM aqueous hydrochloric acid solution was added to the reaction mixture, and the resulting white precipitate (AmpB-Fmoc) was separated by centrifugation. The precipitate was collected and dissolved in DMSO and freeze-dried to give AmpB-Fmoc as a powder. 24.8 mg of AmpB-Fmoc, 8.88 µL of N,N-diisopropylethylamine, and 9.0 mg of 4-(4,6-dimethoxy-1,3,5-triazin-2-yl)-4-methylmorpholinium chloride were dissolved in 4.5 mL of anhydrous dimethylformamide. The mixture was stirred at room temperature for 30 minutes. To this, 12.5 mg of a commercial DBCO-PEG4-amine was added, and the mixture was stirred at 28°C for 5 hours. Fmoc deprotection was performed with addition of 1.2 mL of piperidine to yield AmpB-DBCO, which is represented by the general formula (9). 120 mL of diethyl ether was added to the reaction mixture, and the resulting yellow precipitate (AmpB-DBCO) was separated by centrifugation. The precipitate was collected, dissolved in DMSO, and purified by high performance liquid chromatography (HPLC) using a reverse-phase column (Sunniest RP-AQUA, 5 µm, 250 × 10 mm, ChromaNik Technologies Inc.). The HPLC conditions were as follows. Column temperature: 30°C; Detection: UV absorption at 405 nm; Mobile phase A: 0.1% (v/v) formic acid in water; Mobile phase B: acetonitrile; Gradient of mobile phase: linear gradient of 40% to 100% B in 30 minutes. The acetonitrile was evaporated off from the eluted fraction containing the AmpB-DBCO represented by the general formula (9), and the residue was freeze-dried to give the AmpB-DBCO represented by the general formula (9) as a yellow powder. 366 µg of the AmpB-DBCO represented by the general formula (9) and 689 µg of an ε-PL-PEG-azide containing a 9- to 18-mer ε-PL represented by the general formula (7) were dissolved in an 86% aqueous DMSO solution. The mixture was subjected to click chemistry reaction while stirred at 30°C for 5 hours. The reaction mixture was analyzed by ESI-TOF-MS/HPLC. The results of the analysis confirmed the synthesis of an ε-PL-DBCO-AmpB represented by the general formula (5) (wherein n is an integer of 9 to 17) (Figs. 6A and 6B).

### Water solubility of ε-PL-DBCO-AmpB represented by general formula (5)

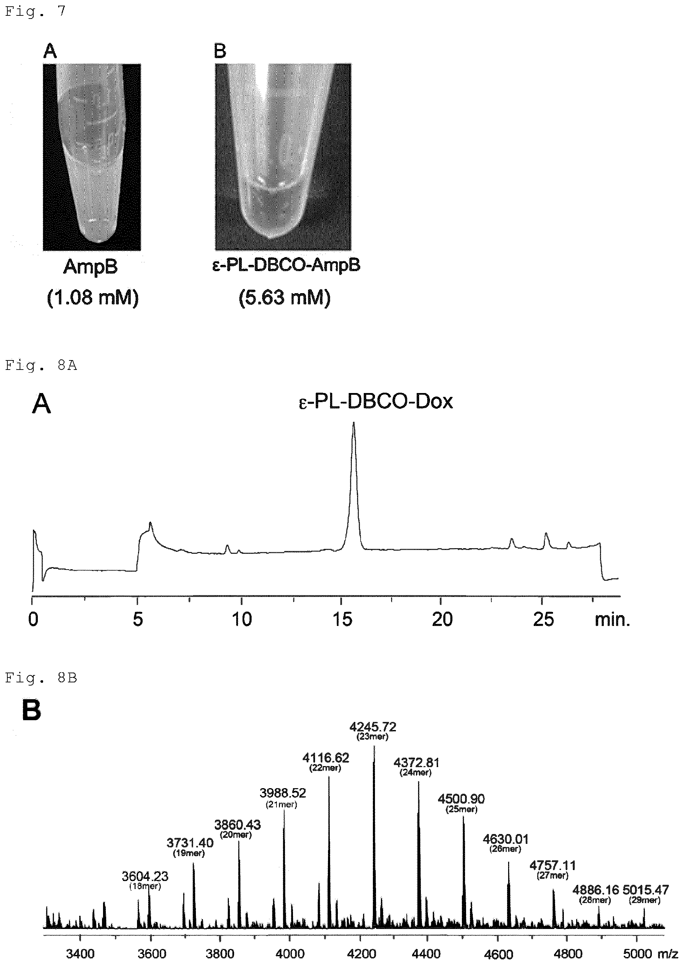

The ε-PL-DBCO-AmpB represented by the general formula (5) (wherein n is an integer of 9 to 17) and the AmpB represented by formula (8) were separately added in water and tested for water solubility. AmpB did not dissolve at all in water at a concentration of 1.08 mM, but the ε-PL-DBCO-AmpB represented by the general formula (5) (wherein n is an integer of 9 to 17) easily dissolved in water at a concentration as high as 5.63 mM, showing high water solubility (Fig. 7). These results show that ε-PL modification can provide the poorly water-soluble AmpB with a high water solubility. Also shown is that the ε-PL-PEG-azide represented by the general formula (10) (wherein n is an integer of 9 to 18) is a useful tool to allow effective and simple ε-PL modification of drugs such as pharmaceutical products.

### Antifungal activity of ε-PL-DBCO-AmpB represented by general formula (5)

The minimum inhibitory concentrations (MICs) of the ε-PL-DBCO-AmpB represented by the general formula (5) (wherein n is an integer of 9 to 17), the AmpB represented by formula (8) and the 9- to 18-mer ε-PL-PEG-azide represented by the general formula (10) (wherein n is an integer of 9 to 18) were measured using *Aspergillus brasiliensis* NBRC9455 as a test fungus. The fungi were cultured in a commercial RPMI-1640 medium at 30°C for 5 days. The MIC of the ε-PL-DBCO-AmpB represented by the general formula (5) was 1.9 µg/mL, the MIC of the AmpB represented by formula (8) was 0.3 µg/mL, and the MIC of the 9- to 18-mer ε-PL-PEG-azide represented by the general formula (10) was 72 µg/mL. The above results show the ε-PL-modified AmpB, i.e., the ε-PL-DBCO-AmpB represented by the general formula (5) retains sufficient antifungal activity as well as being water-soluble. The poorly water-soluble antibiotic AmpB represented by formula (8), due to its poor intestinal absorption in oral administration, is used to treat fungal infections only in gastrointestinal tracts such as the oral cavity and esophagus. However, as shown above, the water-soluble ε-PL-DBCO-AmpB represented by the general formula (5) has an improved biomembrane permeability due to ε-PL modification (polycationic modification), and therefore, can be used for oral administration to treat deep mycosis.

### Example 4

### ε-PL modification of anticancer agent Dox

17.4 mg of Dox (Dox hydrochloride, manufactured by Funakoshi Co., Ltd.), 19.4 mg of DBCO-PEG4-NHS ester, and 4.9 mg of N,N-dimethyl-4-aminopyridine were dissolved in 3 mL of DMSO. The mixture was stirred at room temperature for 4 hours to yield a compound in which DBCO-PEG4 was conjugated to the amino group of the amino sugar moiety of Dox (DBCO-Dox). The obtained DBCO-Dox was purified by high performance liquid chromatography (HPLC) using a reverse-phase column (Sunniest RP-AQUA, 5 µm, 250 × 10 mm, ChromaNik Technologies Inc.). The HPLC conditions were as follows. Column temperature: 35°C; Detection: UV absorption at 254 nm; Mobile phase A: 0.05% (v/v) formic acid in water and 0.05% (v/v) HFBA in water; Mobile phase B: 0.05% (v/v) formic acid in acetonitrile and 0.05% (v/v) HFBA in acetonitrile; Gradient of mobile phase: isocratic with 50% B for 18 minutes. The acetonitrile was evaporated off from the eluted fraction containing DBCO-Dox, followed by ethyl acetate extraction. The extract was concentrated to dryness to give DBCO-Dox. The obtained DBCO-Dox was completely dissolved in 1 mL of chloroform, and the solution was concentrated to dryness again. 10 µmol of DBCO-Dox and 11 µmol of ε-PL-PEG-azide were dissolved in an 80% aqueous DMSO solution. The mixture was subjected to click chemistry reaction while stirred at room temperature for 3 hours. The reaction mixture was analyzed by ESI-TOF-MS/HPLC. The results of the analysis confirmed the synthesis of an ε-PL-DBCO-Dox represented by the general formula (5-2) (wherein n is an integer of 18 to 29) (Figs. 8A and 8B) .

### Cytotoxicity (anticancer activity) of ε-PL-DBCO-Dox represented by general formula (5-2)

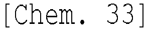

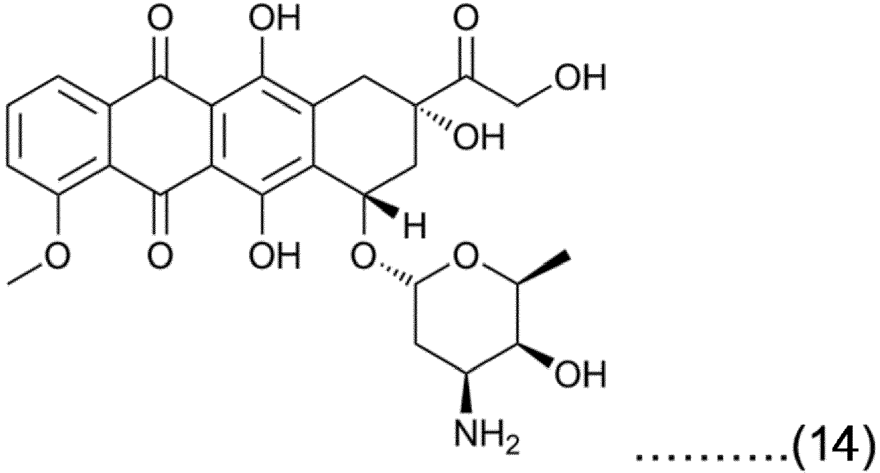

An experiment was performed to examine the cytotoxicity of the Dox represented by formula (14) and the ε-PL-DBCO-Dox represented by the general formula (5-2) (wherein n is an integer of 18 to 29) against human chronic myelogenous leukemia K562 cells and Dox-resistant K562 cells (K562/ADR). The culture of K562 cells and K562/ADR cells was performed in an RPMI1640 medium containing 10% fetal bovine serum (FBS) in an atmosphere of 5% carbon dioxide at 37°C. The cells were suspended at 1 × 10⁵ cells/mL in the medium, and 100 µL (1 × 10⁴ cells) of the cell suspension was seeded on each well of 96-well plates and cultured for 24 hours. Media containing Dox or ε-PL-DBCO-Dox at various concentrations were added to the wells (100 µL/well), and culture was continued for 48 hours. MTT assay was performed to determine cytotoxicity based on viable cell count. As a result, the IC₅₀ values of the Dox represented by formula (14) against K562 cells and K562/ADR cells were 239 ± 72 nM and 1,043 ± 72 nM, respectively. Meanwhile, the IC₅₀ values of the ε-PL-DBCO-Dox represented by the general formula (5-2) against K562 cells and K562/ADR cells were 12.5 ± 1.31 µM and 49.9 ± 9.43 µM, respectively. These results show that the anticancer activity of the ε-PL-DBCO-Dox represented by the general formula (5-2) is about 50 times lower than that of the Dox represented by formula (14), but the ε-PL-DBCO-Dox retains the anticancer activity of the useful anticancer agent doxorubicin and has an improved intestinal absorption and an improved adverse effect profile. In addition, the results indicate that the Dox moiety of the ε-PL-DBCO-Dox can intercalate the double helix structure of DNA. Therefore, only mixing of the ε-PL-DBCO-Dox with plasmid DNAs, gene fragments, or RNAs enables polycationic modification of DNAs or RNAs without covalent bonds. The polycation-modified plasmid DNAs, gene fragments, or RNAs prepared by the method of the present invention can be directly delivered into cells.

### Example 5

### ε-PL modification of high-molecular pharmacological substance

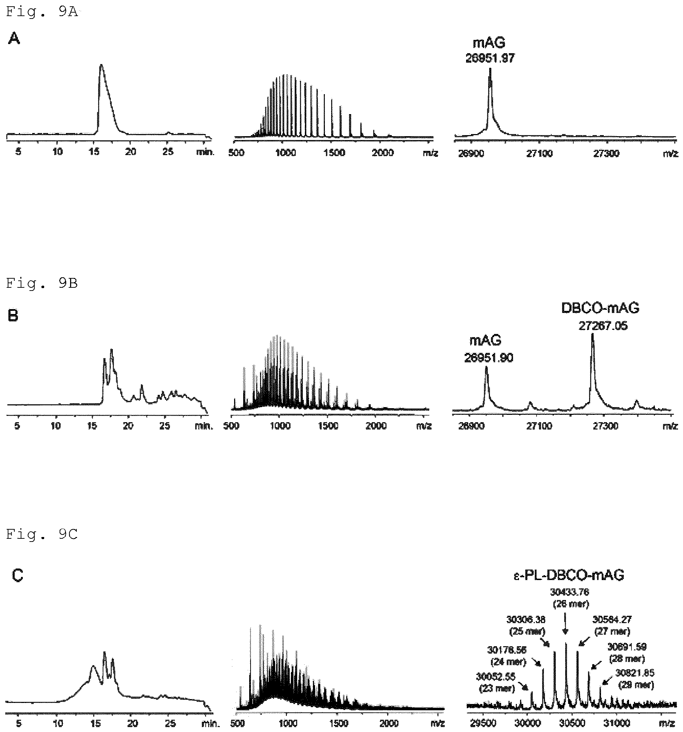

As an exemplary high-molecular pharmacological substance, a fluorescent protein, monomeric Azami-Green (mAG) was subjected to ε-PL modification. A plasmid highly expressing mAG as a C-terminal His-tagged fusion protein (mAG/pET21a) was introduced into *E. coli* BL21 (DE3). The transformed *E. coli* was inoculated into LB medium (containing 100 µg/mL ampicillin) and cultured at 37°C for 2.5 hours. The expression of mAG was induced by 0.1 mM IPTG, and culture was continued at 18°C overnight. The bacterial cells were collected, suspended in a buffer solution (50 mM Tris-HCl, 300 mM NaCl, 10 mM imidazole, pH 7.0), and disrupted ultrasonically. The cell lysate was centrifuged, and the cell-free extract was collected and passed through a Ni affinity column to purify mAG. The purified mAG was dialyzed against 100 mM sodium bicarbonate buffer (pH 9.0) overnight. DBCO-sulfo-NHS was added in three portions (totaling 1 Eq relative to mAG) at hourly intervals at 4°C. After a total reaction time of 3 hours, DBCO-conjugated mAG (DBCO-mAG) was obtained. The obtained DBCO-mAG was dialyzed against 100 mM sodium phosphate buffer (pH 6.0) overnight. 1.1 Eq (relative to DBCO-mAG) of a 20- to 30-mer ε-PL-PEG-azide represented by the general formula (10) (wherein n is an integer of 20 to 30) was added, and reaction was allowed to proceed at 4°C for 1 hour. The reaction mixture was analyzed by ESI-TOF-MS/HPLC. The results of the analysis confirmed the synthesis of an ε-PL-DBCO-mAG represented by the general formula (5-3) (wherein n is an integer of 23 to 29) (Figs. 9A, 9B, and 9C).

### Evaluation of biomembrane permeability of ε-PL-DBCO-mAG

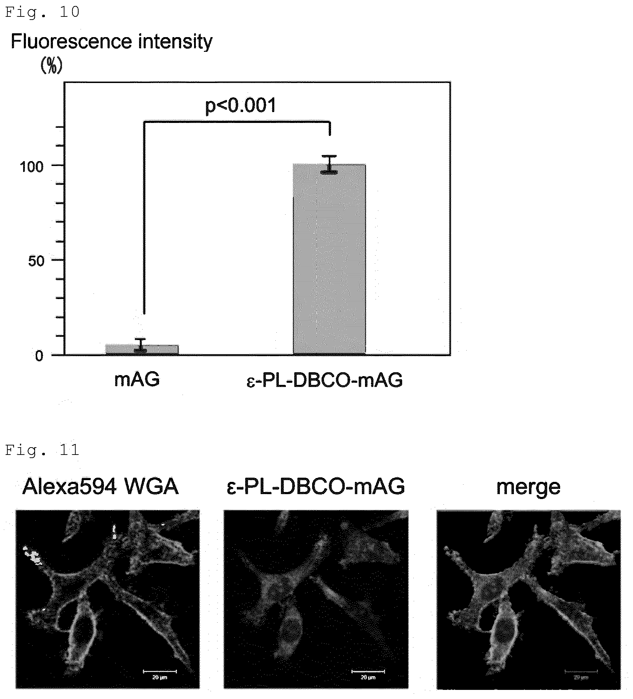

Proteins usually cannot permeate biomembranes such as cell membranes. An experiment was performed to examine the biomembrane permeability of an ε-PL-modified mAG, that is, the ε-PL-DBCO-mAG represented by the general formula (5-3) (wherein n is an integer of 23 to 29) using HeLa cells, which are animal cells. The culture of HeLa cells was performed in a Dulbecco's modified Eagle's medium (DMEM) containing 10% fetal bovine serum (FBS) in an atmosphere of 5% carbon dioxide at 37°C. Under such culture conditions, the cells were cultured in 12-well plates until grown to 70 to 80% confluency. The cells were washed twice with DMEM without FBS (DMEM w/o FBS). DMEM w/o FBS was added to the cells, and after cooling to 4°C, mAG or the ε-PL-DBCO-mAG represented by the general formula (5-3) was added at 50 µM. Incubation was performed at 4°C for 30 minutes. The cells were washed successively with phosphate buffered saline (PBS) twice, with PBS containing heparin (100 µg/mL), and with PBS twice. To the cells, 300 µL of 100 mM Tris-HCl buffer solution (pH 8.0) containing 1% Triton X-100 was added for extraction of the compound taken up by the cells. After removing the insoluble fraction by centrifugation, fluorescence intensity was measured. As a result, the ε-PL-DBCO-mAG represented by the general formula (5-3) had an about 10 times higher biomembrane permeability than that of the mAG represented by the general formula (5-3)'' (Fig. 10). By confocal laser scanning microscopy, the ε-PL-DBCO-mAG represented by the general formula (5-3) was visually observed to be present in the cells, partially in the nuclei (Fig. 11) .

### Example 6

### Chemical stability test of ε-PL-PEG-alkyne represented by general formula (11)

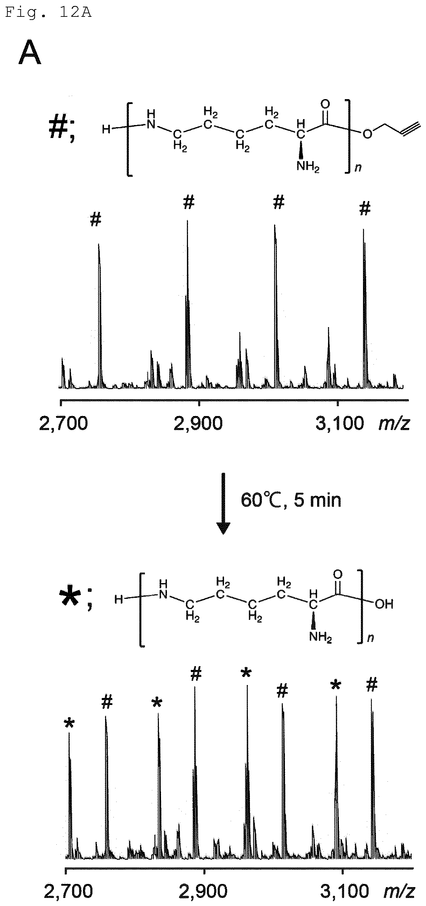

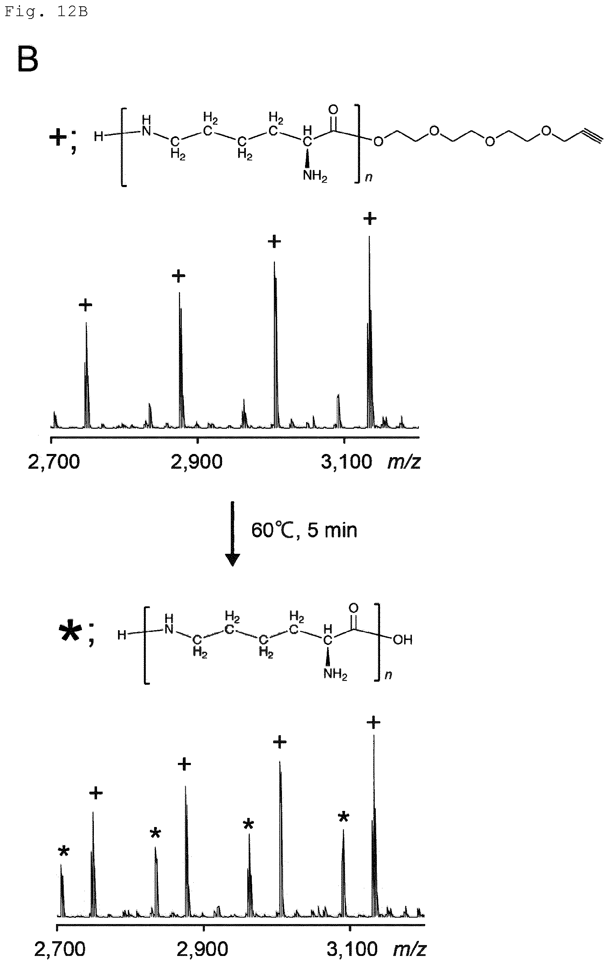

An experiment was performed to examine the chemical stability of the ester bond of the ε-PL-PEG-alkyne represented by the general formula (11) (wherein n is an integer of 17 to 26) obtained in Example 1 and an ε-PL-alkyne represented by the general formula (15) (wherein n is an integer of 17 to 26) obtained as described in Example 1. The ε-PL-PEG-alkyne and the ε-PL-alkyne were separately dissolved at a concentration of 1 mM in Tris-HCl buffer solution (pH 8.0), heated at 60°C for 5 minutes, and analyzed by ESI-TOF-MS/HPLC. The results show that the ester bond of the ε-PL-PEG-alkyne is less prone to hydrolysis and more chemically stable as compared with the ε-PL-alkyne (Figs. 12A and 12B).

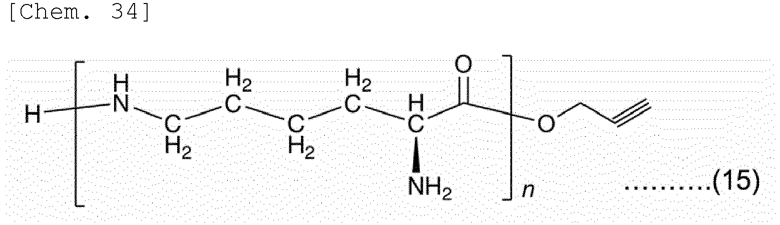

### INDUSTRIAL APPLICABILITY

The present invention not only provides a polycationic derivative which is intended for use in chemical attachment to a pharmacological substance to improve the biomembrane permeability of the pharmacological substance, but also provides a pharmacological substance which has the polycationic derivative attached thereto and thus has an improved biomembrane permeability.

## Claims

1. A compound having a structure represented by the general formula (1): 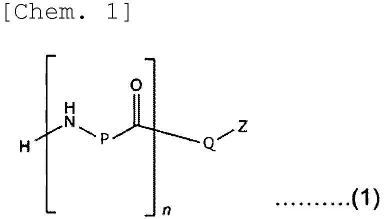 (wherein
n is 2 to 100;
P represents a (C₁-C₅) alkylene group substituted by an amino group;
Q is a structure represented by the following general formula (2): 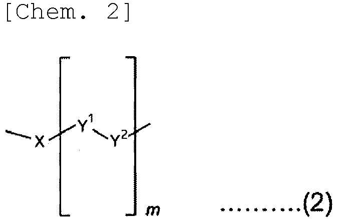 (wherein X represents an oxygen atom, an imino group, a sulfur atom, NHCO, or CONH; Y¹ and Y² may be the same or different and each represent an alkylene group of 1 to 6 carbon atoms, an oxygen atom, or an imino group; and m is an integer of 1 to 30) or the following general formula (3): 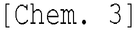 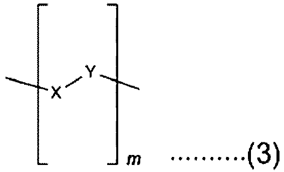 (wherein X represents an oxygen atom, an imino group, a sulfur atom, NHCO, or CONH; Y represents an alkylene group of 1 to 6 carbon atoms, an oxygen atom, or an imino group; and m is an integer of 1 to 30); and
Z represents a click functional group) or
a salt thereof.

2. The compound or the salt thereof according to claim 1, wherein the compound is represented by the general formula (4): 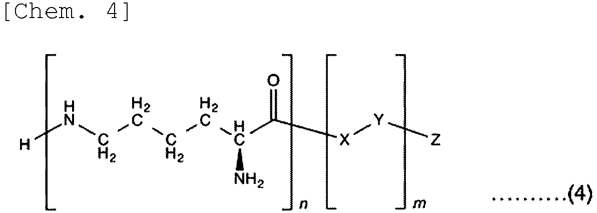 (wherein
n is an integer of 2 to 100;
m is an integer of 1 to 30;
X represents an oxygen atom or an imino group;
Y represents an alkylene group of 1 to 6 carbon atoms; and
Z represents a click functional group).

3. The compound or the salt thereof according to claim 1 or 2, wherein the click functional group is an azido group, an alkenyl group, an alkynyl group, a nitrile group, a thiol group, a maleimide group, an epoxide group, an aziridine group, a thiirane group, or a triazole group.

4. A method for producing the compound or the salt thereof according to claim 1, the method comprising the steps of:
culturing a microbe capable of producing ε-poly-L-lysine (ε-PL), ε-poly-D-lysine, ε-poly-L-β-lysine, ε-poly-D-β-lysine, δ-poly-L-ornithine, δ-poly-D-ornithine, δ-poly-L-β-ornithine, δ-poly-D-β-ornithine, γ-poly-L-diaminobutanoic acid, γ-poly-D-diaminobutanoic acid, β-poly-L-diaminopropionic acid, or β-poly-D-diaminopropionic acid, wherein the culture is performed in the presence of a compound represented by the general formula (2a): 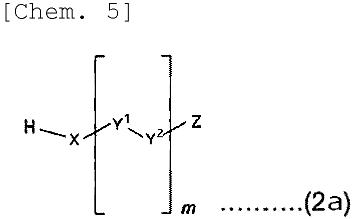 (wherein X, Y¹, Y², Z, and m are as defined in the above general formula (1)) or
the general formula (3a): 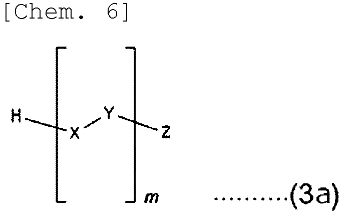 (wherein X, Y, Z, and m are as defined in the above general formula (1)) or
a salt thereof; and
harvesting the compound or the salt thereof according to claim 1 produced in the previous step.

5. A method for producing the compound or the salt thereof according to claim 2, the method comprising the steps of:
culturing a microbe capable of producing ε-PL, wherein the culture is performed in the presence of a compound represented by the general formula (3a): 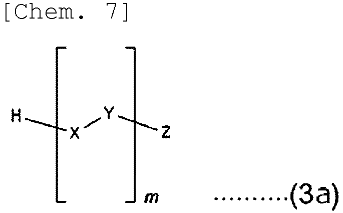 (wherein X represents an oxygen atom or an imino group, Y represents an alkylene group of 1 to 6 carbon atoms, Z represents a click functional group, and m represents an integer of 1 to 30) or
a salt thereof; and
harvesting the compound or the salt thereof according to claim 2 produced in the previous step.

6. A pharmacological substance having the compound according to claim 1 or a pharmaceutically acceptable salt thereof attached thereto by a chemical bond.

7. A pharmacological substance having a compound represented by the general formula (4): 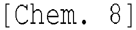 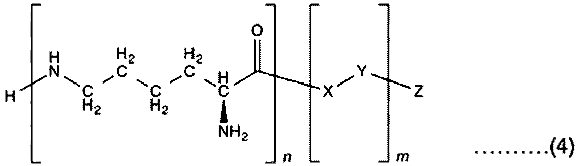 (wherein
n is an integer of 2 to 100;
m is an integer of 1 to 30;
X represents an oxygen atom and an imino group;
Y represents an alkylene group of 1 to 6 carbon atoms; and
Z represents a click functional group) or
a pharmaceutically acceptable salt thereof attached thereto by a chemical bond.

8. The substance according to claim 6 or 7, wherein the pharmacological substance is an antimicrobial agent, an antibacterial agent, or an anticancer agent.

9. The substance according to claim 8,
wherein the antimicrobial agent is (A) an antifungal agent;
wherein the antibacterial agent is (B) a β lactam antibiotic, (C) an aminoglycoside antibiotic, (D) a tetracycline antibiotic, (E) a peptide antibiotic, or (F) an antibacterial antibiotic that can form a bond with the click functional group through an organic chemical or biochemical process; or
wherein the anticancer agent is (G) an anticancer agent that can form a bond with the click functional group through an organic chemical or biochemical process.

10. The substance according to claim 9,
wherein (A) the antifungal agent is (a-1) a polyene antibiotic selected from amphotericin B, nystatin, trichomycin, and pimaricin; (a-2) micafungin; (a-3) itraconazole; (a-4) ketoconazole; (a-5) fluconazole; (a-6) miconazole; or (a-7) flucytosine,
wherein (B) the β lactam antibiotic is (b-1) penicillin; (b-2) ampicillin; (b-3) talampicillin; (b-4) bacampicillin; (b-5) cephalosporin C; (b-6) cefalexin; (b-7) cefradine; (b-8) cephamycin; or (b-9) imipenem,
wherein (C) the aminoglycoside antibiotic is (c-1) gentamicin; (c-2) kanamycin; (c-3) tobramycin; (c-4) amikacin; (c-5) arbekacin; or (c-6) ribostamycin,
wherein (D) the tetracycline antibiotic is (d-1) oxytetracycline; or (d-2) tetracycline,
wherein (E) the peptide antibiotic is (e-1) bacitracin; (e-2) gramicidin; (e-3) polymyxin B; (e-4) vancomycin; or (e-5) teicoplanin,
wherein (F) the antibacterial antibiotic that can form a bond with the click functional group through an organic chemical or biochemical process is an antibacterial antibiotic having an amino group, an imino group, a carboxyl group, a hydroxy group, a carbonyl group, a thiol group, a phosphate group, an aldehyde group, or the like, or
wherein (G) the anticancer agent that can form a bond with the click functional group through an organic chemical or biochemical process is an anticancer agent having an amino group, an imino group, a carboxyl group, a hydroxy group, a carbonyl group, a thiol group, a phosphate group, an aldehyde group, or the like, for example, (f-1) actinomycin D; (f-2) mitomycin; (f-3) an anthracycline antineoplastic agent selected from doxorubicin, daunorubicin, and aclarubicin; (f-4) bleomycin; or (f-5) cisplatin.

11. The substance according to claim 6 or 7, wherein the pharmacological substance is a protein or a gene.

12. The substance according to claim 11, wherein the protein or gene is a protein or gene that can form a bond with the click functional group through an organic chemical or biochemical process.

13. A method for producing the pharmacological substance according to claim 7, the method comprising attaching the compound represented by the general formula (4) or the pharmaceutically acceptable salt thereof specified in claim 7 to a pharmacological substance by a chemical bond.

14. The substance according to any one of claims 7 to 10, wherein the substance is an amphotericin B modified with ε-PL by click chemistry and is represented by the general formula (5): 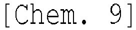 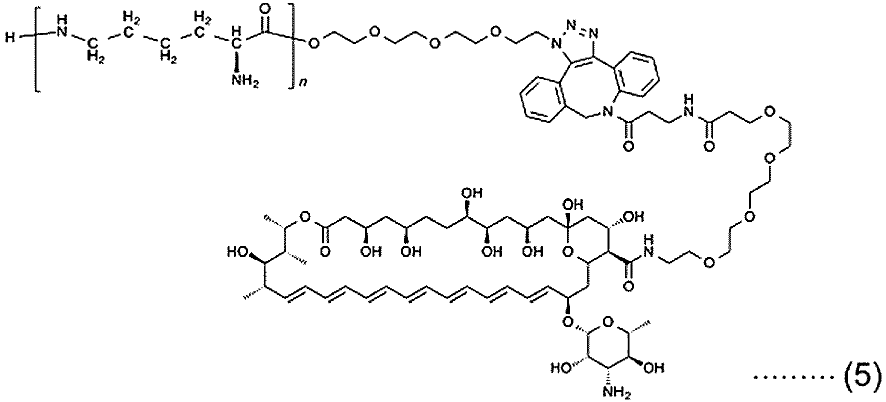 (wherein n is an integer of 2 to 100).

15. The substance according to any one of claims 7 to 10, wherein the substance is a doxorubicin modified with ε-PL by click chemistry and is represented by the general formula (5-2): 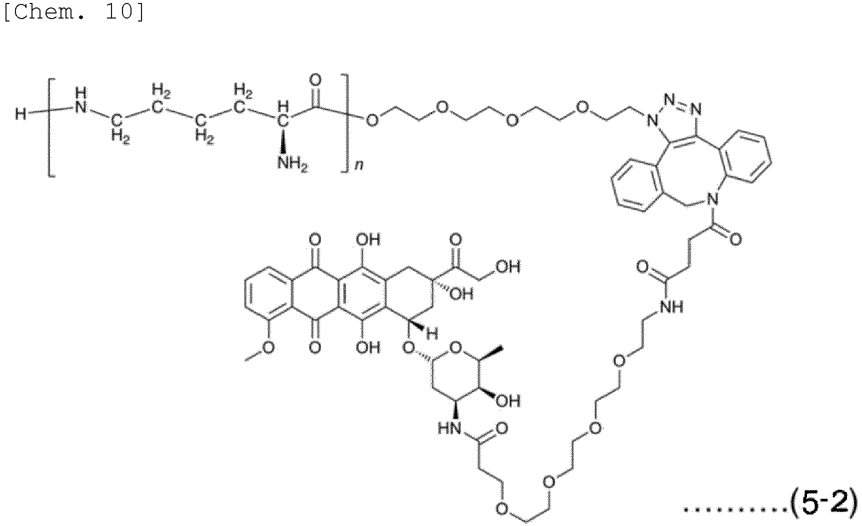 (wherein n is an integer of 2 to 100).

16. The substance according to any one of claims 7 to 10, wherein the substance is a fluorescent protein monomeric Azami-Green modified with ε-PL by click chemistry and is represented by the general formula (5-3): 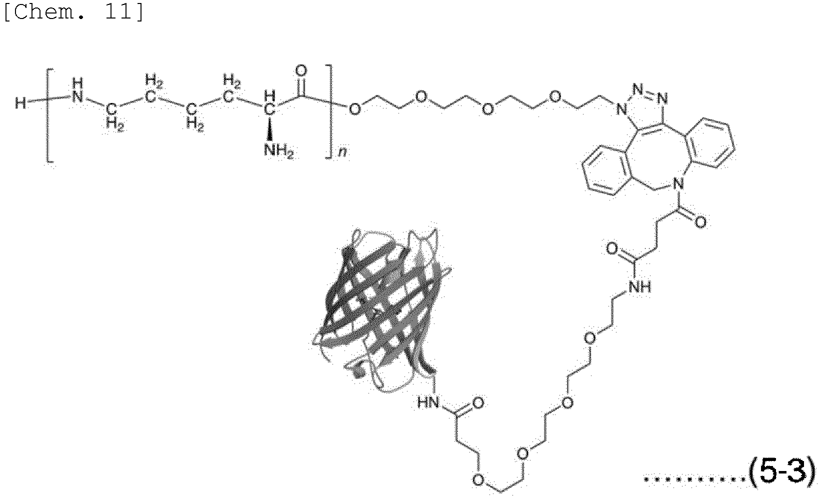 (wherein n is an integer of 2 to 100, and a group represented by formula (5-3)': 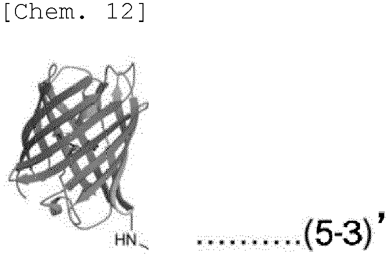 is formed by removing one hydrogen atom from a fluorescent protein monomeric Azami-Green represented by formula (5-3)'' :

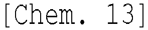

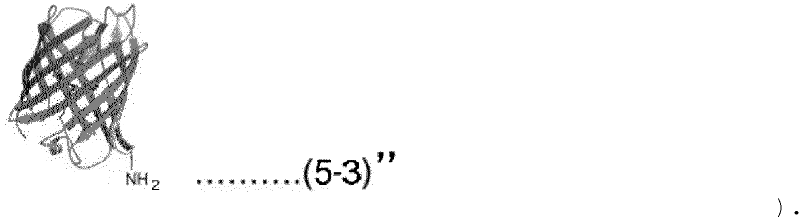

17. Use of a compound represented by the general formula (2a) or (3a) or a salt thereof for production of the compound represented by the general formula (1) or the salt thereof according to claim 1.

18. Use of a compound represented by the general formula (3a) : 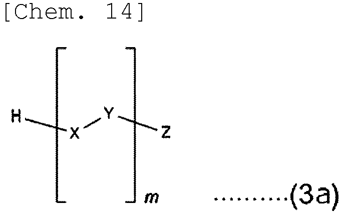 (wherein X represents an oxygen atom or an imino group, Y represents an alkylene group of 1 to 6 carbon atoms, Z represents a click functional group, and m represents an integer of 1 to 30) or
a salt thereof for production of the compound represented by the general formula (4) or the salt thereof specified in claim 7.

19. A method for improving biomembrane permeability of a pharmacological substance, the method comprising attaching a compound represented by the general formula (1) or a salt thereof to the pharmacological substance.

20. Use of a compound represented by the general formula (1) or a salt thereof for production of a pharmacological substance having an improved biomembrane permeability.

21. A method for improving water solubility of a pharmacological substance, the method comprising attaching a compound represented by the general formula (1) or a salt thereof to the pharmacological substance.

22. Use of a compound represented by the general formula (1) or a salt thereof for production of a pharmacological substance having an improved water solubility.
